(19) Europäisches Patentamt
European Patent Office
Office européen des brevets

(11) Veröffentlichungsnummer : **0 016 418**
**B1**

(12) # EUROPÄISCHE PATENTSCHRIFT

(45) Veröffentlichungstag der Patentschrift : **16.02.83**

(51) Int. Cl.³ : **C 09 B 57/02**, C 07 D311/58

(21) Anmeldenummer : **80101286.5**

(22) Anmeldetag : **13.03.80**

(54) **Wasserunlösliche Iminocumarinfarbstoffe, Verfahren zu ihrer Herstellung und ihre Verwendung.**

(30) Priorität : **17.03.79 DE 2910592**

(43) Veröffentlichungstag der Anmeldung : **01.10.80 Patentblatt 80/20**

(45) Bekanntmachung des Hinweises auf die Patenterteilung : **16.02.83 Patentblatt 83/07**

(84) Benannte Vertragsstaaten : **DE FR GB IT**

(56) Entgegenhaltungen :
**DE A 2 005 933**
**DE A 2 717 599**
**FR A 2 141 869**
**FR A 2 186 513**
**FR A 2 192 147**
**FR A 2 319 686**

**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt/Main 80 (DE)**

(72) Erfinder : **Engelmann, Axel, Dr.**
**Loreleistrasse 46**
**D-6230 Frankfurt am Main 80 (DE)**

EP 0 016 418 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Wasserunlösliche Iminocumarinfarbstoffe, Verfahren zu ihrer Herstellung und ihre Verwendung

Die vorliegende Erfindung betrifft neue, wasserunlösliche Iminocumarinfarbstoffe der allgemeinen Formel (1)

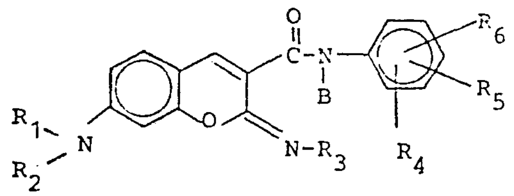

(1)

in welcher B ein Wasserstoffatom oder eine gegebenenfalls substituierte Alkylgruppe bedeutet, $R_1$ und $R_2$ unabhängig voneinander Wasserstoffatome, gegebenenfalls substituierte Alkyl- oder Arylalkylgruppen, ferner Allyl- oder Cycloalkylgruppen mit 3 bis 8 Kohlenstoffatomen, oder zusammen eine Alkylengruppe von 4 bis 5 Kohlenstoffatomen und damit zusammen mit dem Stickstoffatom einen heterocyclischen Ring, welcher weitere Heteroatome enthalten kann, bedeuten, $R_3$ ein Wasserstoffatom oder eine gegebenenfalls substituierte Aryl-, Alkylcarbonyl-, Arylcarbonyl-, Aryl-alkenylen-carbonyl-, Alkoxycarbonyl-, Cycloalkoxycarbonyl-, Aryloxycarbonyl-, Alkylcarbamoyl-, Phenylcarbamoyl-, Alkylsulfonyl- oder Arylsulfonylgruppen darstellt, $R_4$, $R_5$ und $R_6$ unabhängig voneinander Wasserstoff- oder Halogenatome, gegebenenfalls substituierte Alkyl-, Alkoxy-, Carbonsäurealkylester-, Sulfonylalkyl-, Dialkylamino-, Fluoralkylgruppen, ferner Cyan- oder Nitrogruppen bedeuten, und $R_4$ und $R_5$ zusammen, falls in o-Stellung zueinander stehend, die Gruppierung $-(CH{=}CH)_2-$ darstellen.

Die Erfindung betrifft insbesondere die neuen, wasserunlöslichen Iminocumarinfarbstoffe der allgemeinen Formel (2)

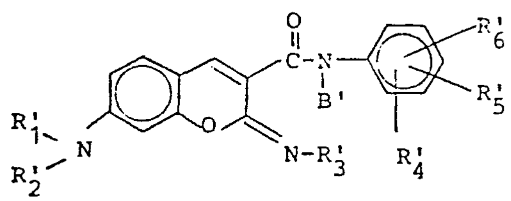

(2)

in welcher B' ein Wasserstoffatom oder eine $Alkyl_{C_1-C_4}$gruppe, $R_1'$ und $R_2'$ unabhängig voneinander Wasserstoffatome, $Alkyl_{C_1-C_4}$ gruppen, welche durch Chlor- oder Bromatome, $Alkoxy_{C_1-C_4}$, Cyan-, Hydroxyl-, Acetoxy-, Propionyloxy- oder Phenoxygruppen substituiert sein können, oder Allyl-, Cyclopentyl-, Cyclohexyl-, Phenyl-, Benzyl- oder Phenäthylgruppen bedeuten, oder zusammen mit dem Stickstoffatom einen Pyrrolidin-, Piperidin-, Pyrazolin-, Piperazin- oder Morpholinring bilden, $R_3'$ ein Wasserstoffatom od. eine Phenyl-, $-CO-Alkyl_{C_1-C_4}$, $-CO-Alkoxy_{C_1-C_4}$, —CO-Cyclohexyloxy-, —CO-Phenyl-, —CO-Phenoxy-, —CO-CH=CH-Phenyl-, $-CO-NH-Alkyl_{C_1-C_4}$, —CO-NH-Phenyl-, $-SO_2-Alkyl_{C_1-C_4}$ oder $-SO_2$-Phenylgruppe bedeuten, welche im Alkyl-, Alkoxy- oder Phenylrest durch Fluor-, Chlor- oder Bromatome oder Trifluormethyl-, $Alkyl_{C_1-C_4}$, $Alkoxy_{C_1-C_4}$, Cyan- oder Nitrogruppen substituiert sein können und $R_4'$, $R_5'$ und $R_6'$ unabhängig voneinander Wasserstoff-, Fluor-, Chlor- od. Bromatome, $Alkyl_{C_1-C_4}$, $Alkoxy_{C_1-C_4}$, Trifluormethyl-, Cyano-, Nitro-, $-N(alkyl_{C_1-C_4})_2$, $-\underset{\underset{O}{\parallel}}{C}-O-Alkyl_{C_1-C_4}$ oder $-SO_2-Alkyl_{C_1-C_4}$ gruppen bedeuten, und $R_4'$ und $R_5'$ zusammen, falls in o-Stellung zueinander stehend, die Gruppierung $-(CH = CH)_2-$ darstellen können.

Die Substituenten $R_1$ und $R_2$ in der vorstehend genannten Formel (1) bzw. die Substituenten $R_1'$ und $R_2'$ in der vorstehend genannten Formel (2) stellen beispielsweise die Methyl-, n- oder i-Propyl-, n- oder i-Butylgruppe oder vorzugsweise die Äthylgruppe dar. An substituierten Alkylgruppen für $R_1$, $R_2$, $R_1'$ und $R_2'$ kommen beispielsweise die β-Methoxyäthyl-, β-Cyanäthyl-, β-Chloräthyl-, β-Acetoxäthyl- oder γ-Methoxypropylgruppe in Betracht. Geeignete Arylgruppen sind beispielsweise die Phenylgruppe, geeignete Arylalkylreste beispielsweise die Benzyl- oder Phenäthylgruppe. Als Cycloalkylreste kommen beispielsweise die Cyclopentyl- oder Cyclohexylgruppe in Frage. Zusammen mit dem Stickstoffatom können die in Rede stehenden Substituenten einen heterocyclischen Ring, wie beispielsweise den Pyrrolidin-, Piperidin-, Morpholin-, Piperazin- oder N-Methylpiperazinring bilden.

Geeignete Gruppen für $R_3$ bzw. $R_3'$ sind beispielsweise die Phenylgruppe, welche durch Chloratome, Methyl-, Trifluormethyl-, Methoxy-, Cyan- oder Carbonsäuremethylestergruppen substituiert sein kann, ferner die Methoxycarbonyl-, Äthoxycarbonyl-, β-Methoxyäthoxycarbonyl-, Propoxycarbonyl-, Cyclohexyloxycarbonyl-, Phenoxycarbonyl-, Acetyl-, Chloracetyl-, Propionyl-, n- und i-Butyryl-, Benzoyl-, Toluyl-, 4-Chlor-benzoyl-, 3-Nitrobenzoyl-, Methansulfonyl-, Cyanmethylsulfonyl-, Äthansulfonyl-, 2-Chloräthansulfonyl-, 2-Methoxyäthansulfonyl-, Propansulfonyl-, Benzolsulfonyl-, Tosyl-, 4-Chlorbenzolsulfonyl-,

Äthylaminocarbonyl-, Propylaminocarbonyl-, Cyclohexylaminocarbonyl-, Diäthylaminocarbonyl-, Phenylaminocarbonyl-, 4-Chlorphenylaminocarbonyl-, 3-Methylphenylaminocarbonyl- oder 4-Trifluormethylphenyl-aminocarbonylgruppe.

Die Substituenten $R_4$, $R_5$ und $R_6$ bzw. $R_4'$, $R_5'$ und $R_6'$ können beispielsweise Fluor-, Chlor- oder Bromatome, Methyl-, Trifluormethyl-, Äthyl-, Methoxy-, Äthoxy-, Carbonsäuremethylester-, Carbonsäureäthylester-, Dimethylamino-, Diäthylamino-, Äthylsulfonyl- oder Oxäthylsulfonylgruppen bedeuten.

Die Farbstoffe der genannten Formel (1) können hergestellt werden, indem man Cyanessigarylide der allgemeinen Formel

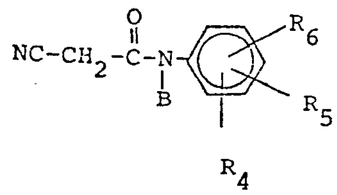

in welcher B, $R_4$, $R_5$ und $R_6$ die vorstehend genannten Bedeutungen haben, mit 4-Aminosalicylaldehyden der allgemeinen Formel

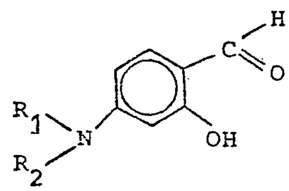

oder deren Estern mit aliphatischen Carbonsäuren mit 1-4 Kohlenstoffatomen, in welcher $R_1$ und $R_2$ die vorstehend genannten Bedeutungen haben, kondensiert und die erhaltenen Iminocumarine der allgemeinen Formel

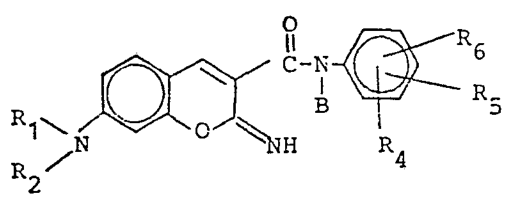

in welcher B, $R_1$, $R_2$, $R_4$, $R_5$ und $R_6$ die vorstehend genannten Bedeutungen haben, mit einem Carbonsäurehalogenid der allgemeinen Formel

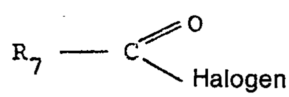

oder mit einem Carbonsäureanhydrid der allgemeinen Formel

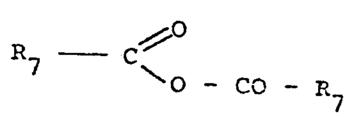

in welchen $R_7$ eine gegebenenfalls substituierte Aryl-, Alkyl-, Alkoxy- oder Aryloxygruppe bedeutet, oder mit Chlorameisensäure-alkyl$_{C_1-C_4}$, -cycloalkyl$_{C_5-C_6}$ oder -phenylester oder mit einem Sulfonsäurehalogenid der allgemeinen Formel

$R_8$—$SO_2$—Halogen

in Gegenwart einer Base acyliert, oder mit einem Isocyanat der allgemeinen Formel

$R_8$—NCO,

worin $R_8$ eine gegebenenfalls substituierte Alkyl- oder Arylgruppe bedeutet, oder mit einem aromatischen

Amin der allgemeinen Formel

$H_2N—Aryl$,

worin die Arylgruppe substituiert sein kann, umsetzt.

Entsprechend können die Farbstoffe der genannten allgemeinen Formel (2) erhalten werden, indem man Cyanessigarylide der allgemeinen Formel

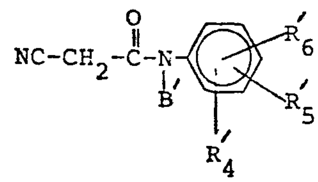

in welcher B', $R_4'$, $R_5'$ und $R_6'$ die vorstehend genannten Bedeutungen haben, mit 4-Aminosalicylaldehyden der allgemeinen Formel

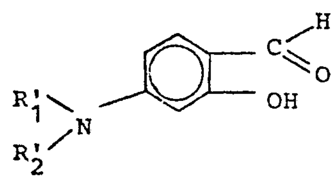

oder deren Estern mit aliphatischen Carbonsäuren mit 1-4 Kohlenstoffatomen, in welchen $R_1'$ und $R_2'$ die vorstehend genannten Bedeutungen haben, kondensiert und die erhaltenen Iminocumarine der allgemeinen Formel

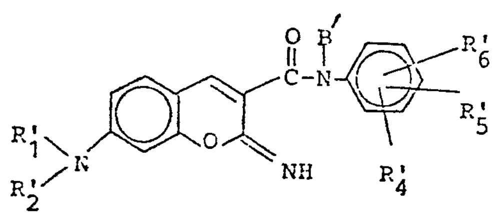

in welcher B', $R_1'$, $R_2'$, $R_4'$, $R_5'$ und $R_6'$ die vorstehend genannten Bedeutungen haben, mit einem Carbonsäurechlorid der allgemeinen Formel

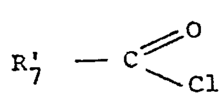

oder mit einem Carbonsäureanhydrid der allgemeinen Formel

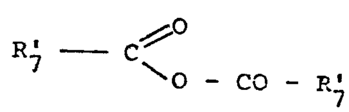

in welcher $R_7'$ eine $Alkyl_{C_1-C_4}$, $Alkoxy_{C_1-C_4}$, Phenyl- oder Phenoxygruppe bedeutet, welche im Alkyl-, Alkoxy- oder Phenylrest durch Fluor-, Chlor- oder Bromatome oder Trifluormethyl-, $Alkyl_{C_1-C_4}$, $Alkoxy_{C_1-C_4}$, Cyan- oder Nitrogruppen substituiert sein können, oder mit Chlorameisensäure-$alkyl_{C_1-C_4}$, -cyclohexyl- oder -phenylester oder mit einem Sulfonsäurechlorid der allgemeinen Formel

$R_8'—SO_2—Cl$

in Gegenwart einer Base acyliert, oder mit einem Isocyanat der allgemeinen Formel

$R_8'—NCO$,

worin $R_8'$ eine $Alkyl_{C_1-C_4}$- oder Phenylgruppe bedeutet, welche durch Fluor-, Chlor- oder Bromatome

oder Trifluormethyl-, Alkyl$_{C_1-C_4}$, Alkoxy$_{C_1-C_4}$, Cyan- oder Nitrogruppen substituiert sein können, oder mit einem aromatischen Amin der allgemeinen Formel

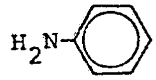

in welcher der Phenylrest durch Fluor-, Chlor- oder Bromatome, Trifluormethylgruppen, Alkyl$_{C_1-C_4}$, Alkoxy$_{C_1-C_4}$, Cyan- oder Nitrogruppen substituiert sein kann, umsetzt.

Die Acylierung der Iminocumarine der vorstehend genannten allgemeinen Formeln mit den Carbonsäurehalogeniden, Chlorameisensäureestern, Carbonsäureanhydriden oder Sulfonsäurehalogeniden erfolgt nach an sich bekannten Verfahren, wie sie beispielsweise in der DE-A-2 234 207 niedergelegt sind.

Die Acylierung nimmt man zweckmäßigerweise in einem Lösungsmittel in Gegenwart einer anorganischen oder organischen Base, vorzugsweise eines Amins, bei Temperaturen zwischen 0 und 160 °C, vorzugsweise zwischen 20 und 100 °C vor.

Als Lösungsmittel eignen sich besonders solche organischer Natur, wie beispielsweise Kohlenwasserstoffe, wie Benzol, Toluol oder Xylol, halogenierte Kohlenwasserstoffe, wie Chloroform oder Chlorbenzol, Äther, wie z. B. Diäthyläther, Glykoldimethyläther, Dioxan oder Tetrahydrofuran oder Aceton.

Geeignete Basen sind beispielsweise Alkalicarbonate, wie Natrium- oder Kaliumcarbonat, Alkalisalze niederer Fettsäuren, wie Natrium- oder Kaliumacetat und ganz besonders organische Basen, wie Triäthylamin, Pyridin oder N,N-Dimethylanilin. Die organischen Basen können gleichzeitig auch als Lösungsmittel dienen.

Als geeignete Säureanhydride seien beispielsweise Acetanhydrid oder Propionsäureanhydrid genannt, als Säurehalogenide beispielsweise Acetylchlorid, Chloracetylchlorid, Benzoylchlorid, Methylbenzoylchlorid, 3-Nitrobenzoylchlorid, 3,5-Dinitrobenzoylchlorid, 4-Chlorbenzoylchlorid, Zimtsäurechlorid, Methansulfochlorid, Äthansulfochlorid, Benzolsulfochlorid, Toluolsulfochlorid, 3-Methoxypropansulfochlorid, Cyanmethansulfochlorid, Chloräthansulfochlorid, Chlorameisensäuremethylester, Chlorameisensäurephenylester, Chlorameisensäure-β-methoxyäthylester, Chlorameisensäurediäthylamid.

Die Umsetzung der weiter oben genannten Iminocumarine mit Isocyanaten, gegebenenfalls in einem inerten, wasserfreien Lösungsmittel, wie beispielsweise Toluol, Chloroform oder Dioxan erfolgt bei Temperaturen zwischen 0 und 150 °C, vorzugsweise zwischen 50 und 100 °C.

Die Umsetzung der weiter oben genannten Iminocumarine mit aromatischen Aminen erfolgt nach Methoden, wie sie beispielsweise in der DE-A-2 226 211 niedergelegt sind. So erhitzt man Iminocumarine beispielsweise mit einem Anilinderivat auf Temperaturen von 50-200 °C, vorzugsweise von 80-150 °C, gegebenenfalls in Gegenwart eines Lösungsmittels, wobei auch das Anilinderivat selbst als Lösungsmittel dienen kann. Weitere Lösungsmittel sind beispielsweise Alkohole, wie Propanol, Butanol, Glykole, sowie deren Mono- und Diäther, vorzugsweise Glycolmonoäthyläther, Dimethylformamid, N-Methylpyrrolidon oder Dimethylsulfoxid. Kohlenwasserstoffe, wie Toluol und Xylol, sowie Chlorkohlenwasserstoffe, wie Chlorbenzol oder Perchloräthylen, kommen ebenfalls in Betracht.

Die Kondensation der Cyanessigarylide der weiter oben genannten allgemeinen Formel mit den 4-Aminosalicyl-aldehyden der weiter oben genannten allgemeinen Formel zu den entsprechenden Iminocumarinen unter den Bedingungen der Knoevenagel-Reaktion gelingt auf einfache Weise und in sehr guter Ausbeute.

Die Cyanessigsäurearylide werden mit dem Dialkylaminosalicylaldehyd oder beispielsweise 2-Acetoxy-4-diäthylaminobenzaldehyd in einem polaren, wassermischbaren Lösungsmittel, wie beispielsweise Alkohol, Glykol, Dioxan, Pyridin, Dimethylformamid, N-Methylpyrrolidon, Dimethylsulfoxid, Glykoldimethyläther unter Zusatz eines Katalysators auf Temperaturen zwischen 50° und 160 °C erwärmt.

Als Katalysatoren dienen beispielsweise Ammoniumacetat, Natronlauge, Alanin oder vorzugsweise Piperidin. Wenn die Reaktionsprodukte nicht aus den Reaktionslösungen ausfallen, werden sie durch Zusatz von Wasser gefällt und durch Filtration isoliert.

Man kann auch die in situ erzeugten Aldehyde unter Vermeidung von Verlusten bei der Isolierung direkt verwenden. So gelingt es, das 3-Diäthylaminophenylacetat in Dimethylformamid zu formylieren und, nachdem man das Reaktionsgemisch alkalisch gestellt hat, mit den Cyanessigsäurearyliden zu Iminocumarinen zu kondensieren. Diese fallen mit der gleichen Reinheit an, wie wenn man vom freien Aldehyd ausgeht.

Die neuen Cumarinfarbstoffe der Formeln (1) und (2) sind überwiegend gelbe bis rote kristalline Pulver, die sich in organischen Medien, insbesondere in Lösungsmitteln, wie Alkoholen, Estern, Amiden, niederen Fettsäuren, Äthern und Ketonen, meist mit intensiv gelbgrüner Fluoreszenz lösen.

Die neuen Farbstoffe eignen sich allein od. in Mischung untereinander od. mit anderen Farbstoffen, vorzugsweise in zubereiteter Form, wie beispielsweise in wäßriger Dispersion oder in Lösung in organischen Lösungsmitteln oder in Emulsion oder Dispersion, die neben einem Lösungsmittel oder einem Lösungsmittelgemisch noch Wasser enthalten können, zum Färben oder Bedrucken von synthetischen Fasermaterialien, wie beispielsweise solchen aus Cellulose-di-, 2 1/2- und -triacetat, Polyamiden, wie Poly-Σ-caprolactam oder Poly-hexamethylendiamiradipat, ferner solchen aus Polyurethanen oder Polycarbonaten, insbesondere aber solchen aus linearen Polyestern, wie Polyäthylenterephthalaten.

Die vorstehend genannten synthetischen Fasermaterialien können zum Färben oder Bedrucken auch in Mischungen untereinander oder mit natürlichen Fasermaterialien, wie Cellulosefasern oder Wolle, vorliegen. Ferner können sie zum Färben in verschiedenen Verarbeitungszuständen, beispielsweise als Kammzug, Flocke, Fäden, Gewebe oder Gewirke, vorliegen.

Die Applikation der erfindungsgemäßen Farbstoffe erfolgt in prinzipiell bekannter Weise, in der Regel aus wäßriger Dispersion, kann jedoch auch aus organischen Lösungsmitteln vorgenommen werden. Die Dispergierung der Farbstoffe kann beispielsweise durch Mahlen in Gegenwart eines Dispergiermittels, wie beispielsweise des Kondensationsproduktes aus Formaldehyd und technischem m-Kresol und Natriumbisulfit das mit Schwefelsäure neutral gestellt wurde, vorgenommen werden. Im übrigen richten sich die Färbebedingungen weitgehend nach der Art der vorliegenden synthetischen Fasermaterialien und deren Verarbeitungszustand.

Beispielsweise erfolgt das Färben von geformten Gebilden aus Celluloseacetat in einem Temperaturbereich von 75 bis 85 °C. Cellulosetriacetatfasern werden bei Temperaturen zwischen etwa 90 und 125 °C gefärbt. Das Aufbringen der Farbstoffe auf Polyamidfasermaterialien geschieht im Temperaturbereich zwischen etwa 90 und 120 °C. Für das Färben von Fasermaterialien aus Polyestern benutzt man die hierfür bekannten Methoden, indem man das Fasermaterial in Gegenwart von Carriern, wie o- oder p-Phenylphenol, Methylnaphthalin oder Methylsalicylat, bei Temperaturen von 100 °C bis etwa 130 °C oder aber ohne Anwendung von Carriern bei entsprechend höheren Temperaturen, beispielsweise zwischen 120 und 140 °C färbt. Außerdem kann man auch so verfahren, daß man die Farbstoffe durch Klotzen mit oder ohne Verdickungsmittel, z. B. Tragant-Verdickung, aufbringt und durch Hitzeeinwirkung, beispielsweise durch Dampf oder Trockenhitze während etwa 1/2 bis 30 Minuten bei Temperaturen im Bereich von etwa 100 bis 230 °C fixiert. Das so gefärbte Material wird dann zur Verbesserung der Reibechtheit zweckmäßig von oberflächlich anhaftendem Farbstoff befreit, beispielsweise durch Spülen oder eine reduktive Nachbehandlung. Diese Nachbehandlung erfolgt im allgemeinen bei 60 bis 120 °C in einer wäßrige Natronlauge, Natriumdithionit und ein nichtionogenes Waschmittel, wie beispielsweise ein Äthylenoxyd-Phenol-Additionsprodukt, enthaltenden Flotte.

Zur Färbung der synthetischen Fasermaterialien aus organischen Lösemitteln kann man beispielsweise so verfahren, daß man bei Raumtemperatur oder darüber, vorzugsweise bei etwa 70 bis 130 °C, gegebenenfalls unter Druck, den Farbstoff aus der Lösung auf die Faser aufzeihen läßt oder so, daß man in einer kontinuierlichen Arbeitsweise Gewebe oder Gewirke mit einer Farbstofflösung imprägniert, trocknet und einer kurzzeitigen Hitzeeinwirkung, beispielsweise bei Temperaturen von 180 bis 210 °C, unterwirft. Als Lösemittel für das Ausziehverfahren seien beispielsweise mit Wasser nicht mischbare Lösemittel mit Siedepunkten zwischen 40 und 170 °C genannt, wie etwa die aliphatischen Halogenkohlenwasserstoffe, wie Methylenchlorid, Trichloräthan, Trichloräthylen, Perchloräthylen oder Trifluortrichloräthan. Insbesondere für ein kontinuierliches Färbeverfahren kommen auch mit Wasser mischbare Lösemittel, wie beispielsweise Alkohole oder Dimethylformamid, in Betracht. Die Lösemittel können natürlich auch als Mischungen vorliegen und weitere in Lösemitteln lösliche Hilfsmittel, wie beispielsweise Oxalkylierungsprodukte von Fettalkoholen, Alkylphenolen und Fettsäuren, enthalten.

Zur Herstellung von Drucken auf den synthetischen Fasermaterialien, beispielsweise solchen aus Polyestern, Polyamiden oder Cellulosetriacetat, können die Farbstoffe in Form wasserhaltiger Zubereitungen angewandt werden, die neben dem fein verteilten Farbstoff geeignete Verdickungsmittel enthalten können. Die Fixierung erfolgt beispielsweise nach dem Drucken und Trocknen durch Dämpfen bei Atmosphärendruck oder unter erhöhtem Druck bis zu 2,5 atü während 10 bis 60 Minuten. Ebenso kann man die Fixierung durch die Einwirkung von Heißluft von 160 bis 120 °C während 30 Sekunden bis 10 Minuten bewirken.

Zusätzlich eignen sich die erfindungsgemäßen Farbstoffe zum Schmelzspinnfärben, vorzugsweise von Polyäthylenterephthalat.

Mit den erfindungsgemäßen Farbstoffen der Formeln 1 u. 2 lassen sich die genannten Fasern, Gewebe und Kunststoffmassen zum Teil extrem brillant grünstichig-gelb fluoreszierend färben. Die Färbungen zeichnen sich durch hohe Farbstärke, gutes Aufbau- und Ziehvermögen, sowie durch sehr gute Echtheitseigenschaften, wie Wasch-, Reib-, Sublimier-, Schweiß- und Lichtechtheiten aus.

Gegenüber dem aus Beispiel 4 der DE-A-2 717 599 bekannten Farbstoff der Formel

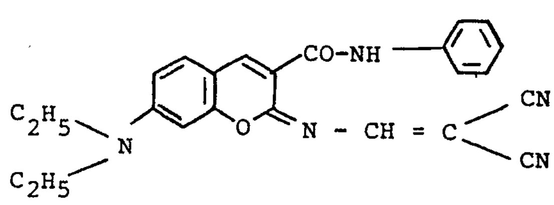

und gegenüber dem aus Beispiel 1 der FR-A-2 319 686 bekannten Farbstoff der Formel

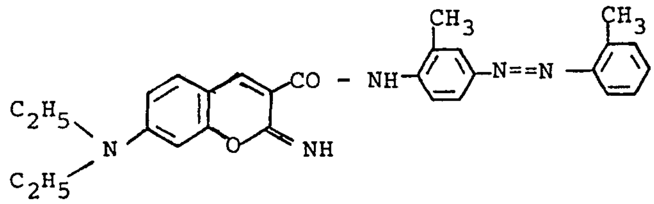

zeichnen sich die erfindungsgemäßen Farbstoffe ähnlicher Struktur durch Anfärben von Polyesterfasern und Wolle im gleichen Farbton aus, wodurch sie zum Färben von Mischgeweben aus den genannten Faserarten geeignet sind, bzw. zum Färben von Polyesterfasermaterialien in brillanten, grünstichig gelben fluoreszierenden Tönen aus.

Die in den folgenden Beispielen angegebenen Teile sind Gewichtsteile.

Beispiel 1

9,7 Teile 4-Diäthylaminosalicylaldehyd und 11,3 Teile 2-Methoxy-5-chlor-cyanessigsäureanilid werden mit 10 Tropfen Piperidin und 200 ml Äthanol am Rückfluß erhitzt, wobei die Verbindung der Formel

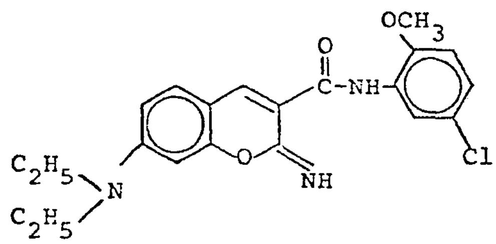

ausfällt.

Nach Abkühlung des Reaktionsgemisches wird der Niederschlag abgesaugt, mit kaltem Äthanol gewaschen und bei 60 °C getrocknet. Gewonnen werden 18 Teile Farbstoff, was einer Ausbeute von 90 % der Theorie entspricht. Der Farbstoff ergibt auf Polyesterfasern grünstichig gelbe Färbungen.

Beispiel 2

11,8 Teile 4-Diäthylamino-2-acetoxybenzaldehyd und 13,2 Teile 2-Chlor-5-trifluormethylcyanessigsäureanilid werden mit 10 Tropfen Piperidin und 200 ml Äthanol 1 Stunde am Rückfluß erhitzt, wobei die Verbindung der Formel

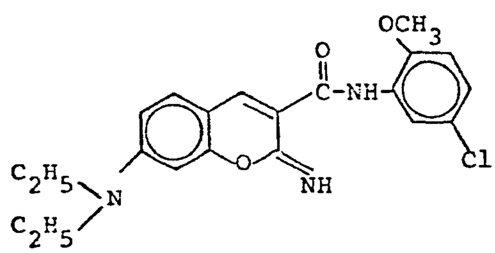

ausfällt. Nach Abkühlung des Reaktionsgemisches wird der Niederschlag abgesaugt, mit kaltem Äthanol gewaschen und bei 60 °C getrocknet. Gewonnen werden 18,7 Teile Farbstoff was einer Ausbeute von 85 % der Theorie entspricht. Der Farbstoff ergibt auf Polyesterfasern grünstichig gelbe Färbungen.

Beispiel 3

8 Teile 7-Diäthylamino-2-iminocumarin-3-carbonsäure-2'-methoxy-5'-chloranilid werden in 50 ml Pyridin, gegebenenfalls unter leichter Erwärmung, gelöst. Man tropft dann bei Raumtemperatur 7,2 Teile Chlorameisensäureäthylester zu und rührt ca. 20 Stunden bei Raumtemperatur. Das Reaktionsgemisch wird nun auf 300 Teile Wasser gegeben und die ausgefallene Verbindung der Formel

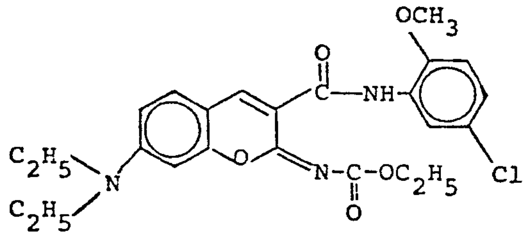

abgesaugt, elektrolytfrei gewaschen und getrocknet. Man erhält 8,5 Teile Farbstoff, was einer Ausbeute von 90 % der Theorie entspricht. Der gewonnene Farbstoff färbt Polyesterfasern in leuchtend grünstichig gelben Farbtönen.

Beispiel 4

8 Teile 7-Diäthylamino-2-iminocumarin-3-carbonsäure-2',5'-dichloranilid werden unter Erwärmung in 50 Teilen Pyridin gelöst. Man läßt unter Rühren abkühlen, gibt 19 Teile 4-Chlorbenzolsulfochlorid hinzu und rührt 24 Stunden bei 60 °C.

Das Reaktionsgemisch wird dann auf 300 Teile Wasser gegeben und die ausgefallene Verbindung der Formel

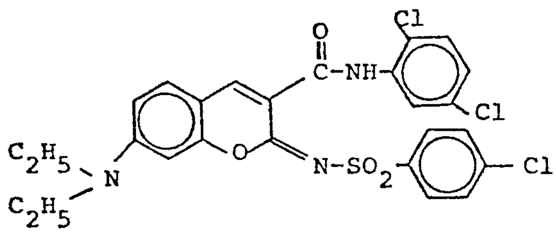

abgesaugt, neutral gewaschen und getrocknet.

Gewonnen werden 8,5 Teile Farbstoff, was einer Ausbeute von 72 % der Theorie entspricht. Der Farbstoff ergibt auf Polyesterfasern eine stark grünstichig gelbe, fluoreszierende Färbung.

Beispiel 5

12 Teile 7-Diäthylamino-2-iminocumarin-3-carbonsäure-2',5'-di-chloranilid werden mit 150 Teilen Chloroform vorgelegt.

Dazu tropft man unter Rühren 15,5 Teile Propionsäureanhydrid, versetzt mit 10 Tropfen konz. Schwefelsäure und erhitzt 24 Stunden zum Sieden. Dann gibt man das Reaktionsgemisch auf 300 ml 2n-Soda-Lösung und saugt den ausgefallenen Farbstoff der Formel

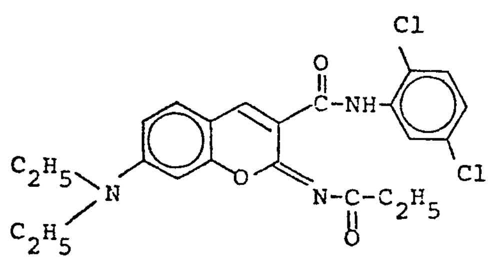

ab. Anschließend wird der Farbstoff neutral gewaschen und getrocknet. Gewonnen werden 9 Teile Farbstoff, was einer Ausbeute von 65 % der Theorie entspricht.

Der Farbstoff ergibt auf Polyesterfasern eine grünstichig gelbe Färbung.

Beispiel 6

4 Teile 7-Diäthylamino-2-iminocumarin-3-carbonsäure-3'-trifluor-methylanilid werden in 150 Teilen trockenem Aceton suspendiert. Dazu gibt man 1,4 Teile fein pulversisiertes, trockenes Kaliumcarbonat. Dann werden 3,5 Teile 90 %iger Chlorameisensäurephenylester zugetropft und das Reaktionsgemisch 20 Stunden bei Raumtemperatur verrührt, bis im Chromatogramm kein Ausgangsmaterial mehr sichtbar ist. Man gibt das Reaktionsgemisch auf 500 Teile Wasser und saugt die ausgefallene Verbindung der Formel

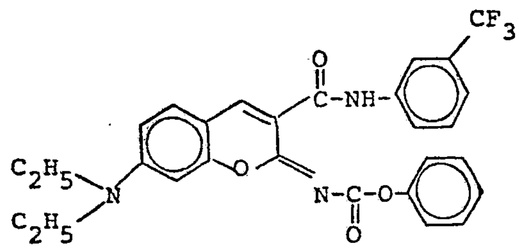

ab. Anschließend wird der Farbstoff elektrolytfrei gewaschen und getrocknet. Gewonnen werden 5 Teile Farbstoff, was einer Ausbeute von 94 % der Theorie entspricht. Der Farbstoff ergibt auf Polyesterfasern leuchtend grünstichig gelbe Färbungen.

Beispiel 7

7,4 Teile Diäthylamino-2-iminocumarin-3-carbonsäure-3'-chloranilid werden gegebenenfalls unter Erwärmen in 70 Teilen Chloroform gelöst. Man gibt 5 Teile Pyridin hinzu, tropft dann 7,3 Teile Chlorameisensäureisopropylester ein. Anschließend wird bei Raumtemperatur gerührt, bis dünnschichtchromatographisch kein Ausgangsmaterial mehr nachweisbar ist. Dann rührt man die Reaktionslösung in 500 Teile Wasser ein, trennt die organische Phase ab und trocknet sie über Natriumsulfat. Nach Abziehen des Lösungsmittels hinterbleiben 9,1 Teile des Farbstoffs der Formel

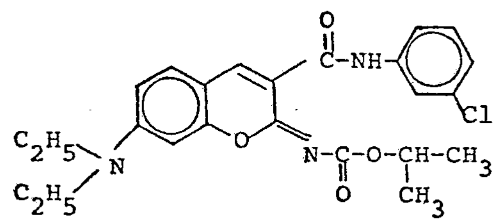

was einer Ausbeute von 94 % der Theorie entspricht. Der Farbstoff ergibt auf Polyesterfasern leuchtend grünstichig gelbe Färbungen.

Beispiel 8

8,5 Teile 7-Diäthylamino-2-iminocumarin-3-carbonsäure-2'-cyan-5'-trifluormethylanilid werden in 70 Teilen Chloroform suspendiert. Man tropft 5 Teile Phenylisocyanat zu und erhitzt 20 Stunden am Rückfluß. Die ausgefallene Verbindung der Formel

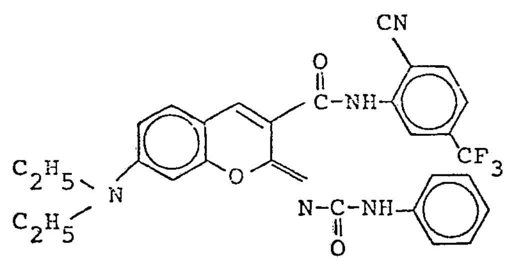

wird abgesaugt, mit eiskaltem Äthanol gewaschen und schließlich getrocknet. Gewonnen werden 9,7 Teile Farbstoff, was einer Ausbeute von 94 % der Theorie entspricht. Der Farbstoff ergibt auf Polyesterfasern sehr brillante, grünstichig gelbe Färbungen.

Beispiel 9

8 Teile 7-Diäthylamino-2-iminocumarin-3-carbonsäure-3'-trifluormethylanilid und 12,7 Teile 4-Chloranilin werden in 50 Teilen Glycolmonoäthyläther etwa 12 Stunden am Rückfluß erhitzt. Nach Abkühlung auf Eistemperatur wird der ausgefallene Farbstoff der Formel

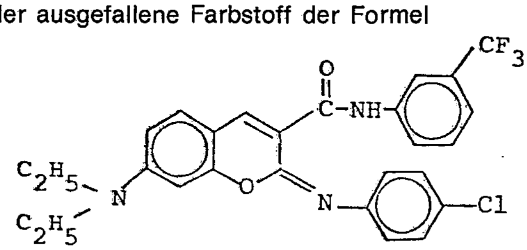

abgesaugt und getrocknet.

Gewonnen werden 6,2 Teile Farbstoff, was einer Ausbeute von 60,5 % der Theorie entspricht. Der Farbstoff ergibt auf Polyesterfasern grünstichig gelbe Färbungen.

## Beispiel 10

20,7 Teile 3-Diäthylaminophenylacetat werden in 30 ml Dimethylformamid vorgelegt. Bei Temperaturen zwischen 40 und 50 °C tropft man 17 Teile $POCl_3$ zu und rührt 5 Stunden bei 60 °C. Unter gelegentlichem Kühlen werden 60 Teile Methanol zugetropft und mit etwa 50 Teilen konzentrierter Natronlauge auf pH 8-9 gestellt. Dann trägt man 26,3 Teile Cyanessigsäure-2-chlor-5-trifluormethylanilid ein, gibt 2 Teile Piperidin hinzu und erhitzt noch 1 Stunde auf 60 °C, wobei man den pH-Wert durch gelegentliche Zugabe von konzentrierter Natronlauge auf 8-9 hält. Nach dem Abkühlen wird die ausgefallene Verbindung der Formel

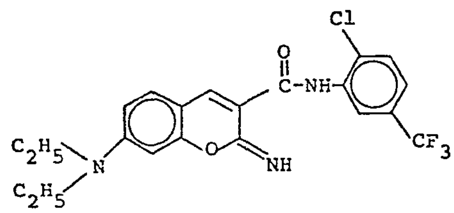

abgesaugt, mit kaltem Äthanol gewaschen und bei 60 °C getrocknet. Gewonnen werden 25 Teile Farbstoff, was einer Ausbeute von 56 % der Theorie entspricht.

In den folgenden Tabellen sind weitere erfindungsgemäße Farbstoffe aufgeführt :

Allgemeine Formel der Farbstoffe der nachstehenden Tabellenbeispiele 11-119 :

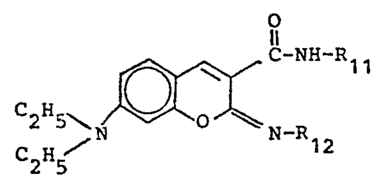

| Beispiel Nr. | $R_{11}$ | $R_{12}$ | Farbton auf Polyesterfasern |
|---|---|---|---|
| 11 | 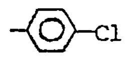 | H | grünstichig gelb |
| 12 | " | 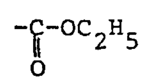 | " |
| 13 | " | 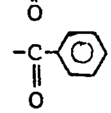 | " |
| 14 | " | 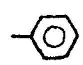 | " |
| 15 | " | $-SO_2-CH_3$ | " |
| 16 | 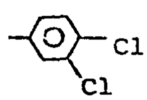 | H | " |
| 17 | " | 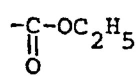 | " |
| 18 | 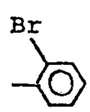 | H | " |

(Fortsetzung)

| Beispiel Nr. | $R_{11}$ | $R_{12}$ | Farbton auf Polyesterfasern |
|---|---|---|---|
| 19 | 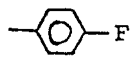 | H | " |
| 20 | " | 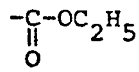 | " |
| 21 | 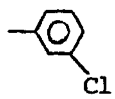 | 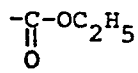 | " |
| 22 | " | $-SO_2CH_3$ | " |
| 23 | 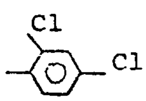 | H | grünstichig gelb |
| 24 | " | 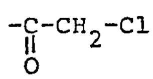 | " |
| 25 | 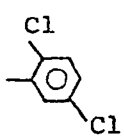 | H | " |
| 26 | " | 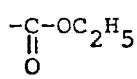 | " |
| 27 | " | 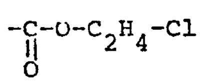 | " |
| 28 | " | 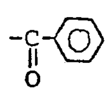 | " |
| 29 | " | 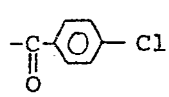 | " |
| 30 | " | 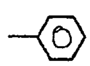 | " |
| 31 | " | 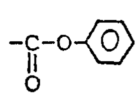 | " |
| 32 | " | 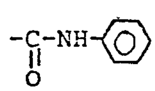 | " |

(Fortsetzung)

| Beispiel Nr. | $R_{11}$ | $R_{12}$ | Farbton auf Polyesterfasern |
|---|---|---|---|
| 33 | " | 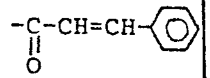 | " |
| 34 | " | $-SO_2CH_3$ | " |
| 35 | 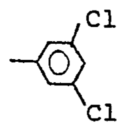 | H | grünstichig gelb |
| 36 | " | $-\underset{\underset{O}{\|}}{C}-OC_2H_5$ | " |
| 37 | " | $-SO_2CH_3$ | " |
| 38 | 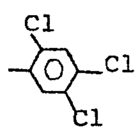 | H | " |
| 39 | " | $-\underset{\underset{O}{\|}}{C}-O-C_4H_9$ | " |
| 40 | " | $-SO_2C_4H_9$ | " |
| 41 | 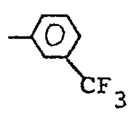 | H | " |
| 42 | " | $-\underset{\underset{O}{\|}}{C}-OC_2H_5$ | " |
| 43 | " | 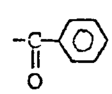 | " |
| 44 | " | 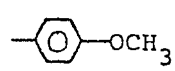 | " |
| 45 | " | 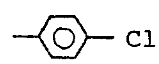 | " |
| 46 | " | 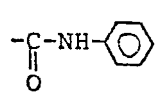 | " |
| 47 | " | $-SO_2-CH_3$ | " |

(Fortsetzung)

| Beispiel Nr. | $R_{11}$ | $R_{12}$ | Farbton auf Polyesterfasern |
|---|---|---|---|
| 48 | 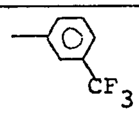 | $-SO_2C_2H_5$ | grünstichig gelb |
| 49 | " | $-SO_2-C_3H_6-OCH_3$ | " |
| 50 | " | 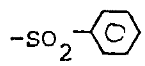 | " |
| 51 | " | 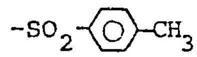 | " |
| 52 | 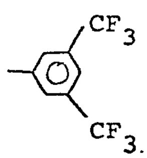 | H | " |
| 53 | " | 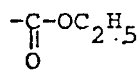 | " |
| 54 | 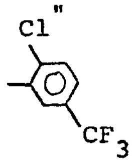 | H | " |
| 55 | " | 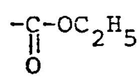 | " |
| 56 | " | 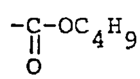 | " |
| 57 | " | 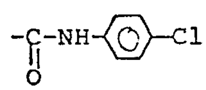 | " |
| 58 | " | $-SO_2-C_2H_4-Cl$ | " |
| 59 | 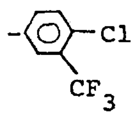 | 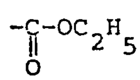 | " |
| 60 | 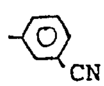 | H | " |
| 61 | 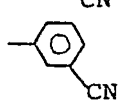 | 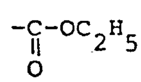 | grünstichig gelb |
| 62 | " | 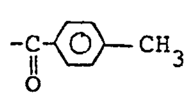 | " |
| 63 | " | $-SO_2CH_3$ | " |
| 64 | 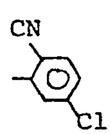 | H | " |

(Fortsetzung)

| Beispiel Nr. | $R_{11}$ | $R_{12}$ | Farbton auf Polyesterfasern |
|---|---|---|---|
| 65 | " | $-\underset{\underset{O}{\|}}{C}-O-C_4H_9$ | " |
| 66 | " | 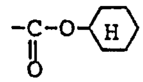 | " |
| 67 | " | $-SO_2C_2H_5$ | " |
| 68 | 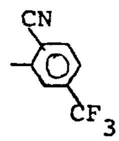 | H | " |
| 69 | " | $-\underset{\underset{O}{\|}}{C}-O-C_2H_5$ | " |
| 70 | " | $-\underset{\underset{O}{\|}}{C}-NH-C_3H_7$ | " |
| 71 | " | 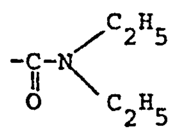 | " |
| 72 | " | 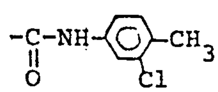 | " |
| 73 | 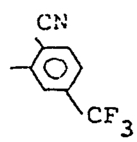 | 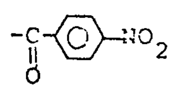 | gelb |
| 74 | " | 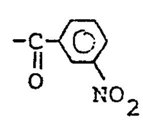 | " |
| 75 | " | $-SO_2-CH_2-CN$ | grünstichig gelb |
| 76 | 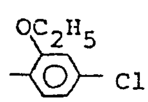 | H | " |
| 77 | " | $-\underset{\underset{O}{\|}}{C}-OC_2H_5$ | " |
| 78 | " | 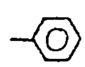 | " |
| 79 | " | $-SO_2CH_3$ | " |
| 80 | 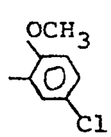 | H | " |

(Fortsetzung)

| Beispiel Nr. | $R_{11}$ | $R_{12}$ | Farbton auf Polyesterfasern |
|---|---|---|---|
| 81 | " | $-\underset{\underset{O}{\|}}{C}-OC_2H_5$ | " |
| 82 | " | $-SO_2CH_3$ | " |
| 83 | 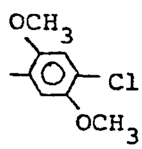 | H | " |
| 84 | " | $-\underset{\underset{O}{\|}}{C}-OC_2H_5$ | " |
| 85 | " | $-SO_2CH_3$ | " |
| 86 | 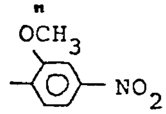 | H | grünstichig gelb |
| 87 | " | $-\underset{\underset{O}{\|}}{C}-OC_2H_5$ | " |
| 88 | " | $-SO_2CH_3$ | " |
| 89 | 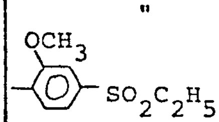 | H | " |
| 90 | " | $-\underset{\underset{O}{\|}}{C}-OC_2H_5$ | " |
| 91 | " | $-SO_2CH_3$ | " |
| 92 | 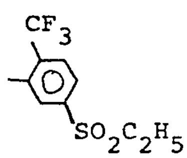 | H | " |
| 93 | " | $-\underset{\underset{O}{\|}}{C}-O-C_3H_7$ | " |
| 94 | " | $-SO_2C_3H_7$ | " |
| 95 | 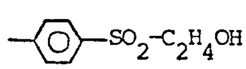 | H | " |
| 96 | 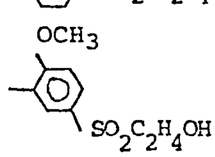 | H | " |
| 97 | 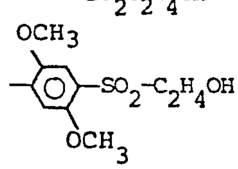 | H | " |

(Fortsetzung)

| Beispiel Nr. | $R_{11}$ | $R_{12}$ | Farbton auf Polyesterfasern |
|---|---|---|---|
| 98 | 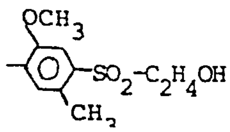 | H | " |
| 99 | 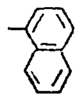 | H | grünstichig gelb |
| 100 | " | 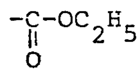 | " |
| 101 | " | $-SO_2CH_3$ | " |
| 102 | 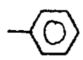 | H | " |
| 103 | " | 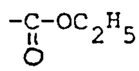 | " |
| 104 | " | 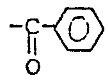 | " |
| 105 | 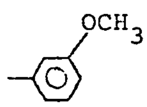 | H | " |
| 106 | " | 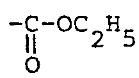 | " |
| 107 | 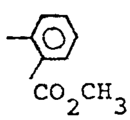 | H | " |
| 108 | " | 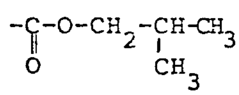 | " |
| 109 | 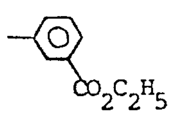 | H | " |
| 110 | " | 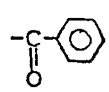 | " |
| 111 | 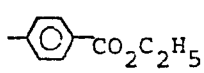 | H | " |
| 112 | " | 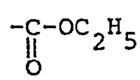 | " |
| 113 | 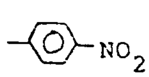 | H | " |
| 114 | 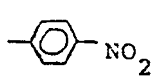 | 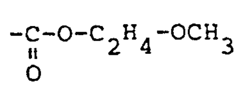 | grünstichig gelb |
| 115 | 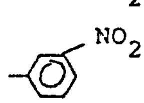 | H | " |

(Fortsetzung)

| Beispiel Nr. | $R_{11}$ | $R_{12}$ | Farbton auf Polyesterfasern |
|---|---|---|---|
| 116 | " | $-\overset{O}{\overset{\|}{C}}-OC_2H_5$ | " |
| 117 | " | $-SO_2C_2H_5$ | " |
| 118 | 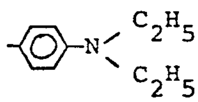 | H | goldgelb |
| 119 | " | $-\overset{O}{\overset{\|}{C}}-OC_2H_5$ | " |
| 120 | " | $-SO_2CH_3$ | " |

Allgemeine Formel der Farbstoffe der nachstehenden Tabellenbeispiele 121-130 :

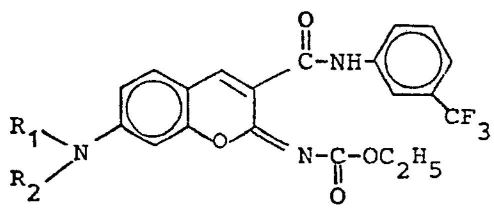

| Beispiel Nr. | $R_1$ | $R_2$ | Farbton auf Polyesterfasern |
|---|---|---|---|
| 121 | $NC-C_2H_4-$ | $CH_3-$ | grünstichig gelb |
| 122 | 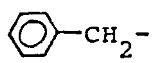 | $CH_3-$ | " |
| 123 | $CH_3O-C_2H_4-$ | $CH_3-$ | " |
| 124 | $CH_3-\overset{O}{\overset{\|}{C}}-O-C_2H_4-$ | $CH_3-$ | " |
| 125 | $H_2C = CH-CH_2-$ | $CH_3-$ | " |
| 126 | $Cl-C_2H_4-$ | $CH_3-$ | " |
| 127 | $C_4H_9$ | $C_4H_9-$ | " |
| 128 | $-C_2H_4-O-C_2H_4-$ | | " |
| 129 | $-CH_2-CH_2-CH_2-CH_2-CH_2-$ | | " |
| 130 | $-C_2H_4-\underset{CH_3}{\underset{\|}{N}}-C_2H_4-$ | | " |

**Ansprüche**

1. Wasserunlösliche Iminocumarinfarbstoffe der allgemeinen Formel (1)

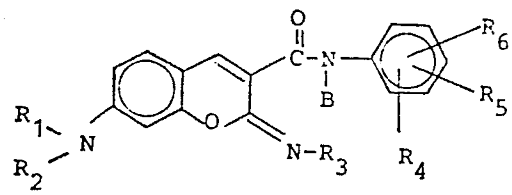

(1)

in welcher B ein Wasserstoffatom oder eine gegebenenfalls substituierte Alkylgruppe bedeutet, $R_1$ und $R_2$ unabhängig voneinander Wasserstoffatome, gegebenenfalls substituierte Alkyl- oder Arylalkylgruppen, ferner Allyl- oder Cycloalkylgruppen mit 3 bis 8 Kohlenstoffatomen, oder zusammen eine Alkylengruppe von 4 bis 5 Kohlenstoffatomen und damit zusammen mit dem Stickstoffatom einen heterocyclischen Ring, welcher weitere Heteroatome enthalten kann, bedeuten, $R_3$ ein Wasserstoffatom oder eine gegebenenfalls substituierte Aryl-, Alkylcarbonyl-, Arylcarbonyl-, Aryl-alkenylen-carbonyl-, Alkoxy-carbonyl-, Cycloalkoxycarbonyl-, Aryloxycarbonyl-, Alkylcarbamoyl-, Phenylcarbamoyl-, Alkylsulfonyl- oder Arylsulfonylgruppe darstellt, $R_4$, $R_5$ und $R_6$ unabhängig voneinander Wasserstoff- oder Halogenatome, gegebenenfalls substituierte Alkyl-, Alkoxy-, Carbonsäurealkylester-, Sulfonylalkyl-, Dialkylamino-, Fluoralkylgruppen, ferner Cyan- oder Nitrogruppen bedeuten, und $R_4$ und $R_5$ zusammen, falls in o-Stellung zueinander stehend, die Gruppierung $—(CH = CH)_2—$ darstellen.

2. Wasserunlösliche Iminocumarinfarbstoffe der allgemeinen Formel (2)

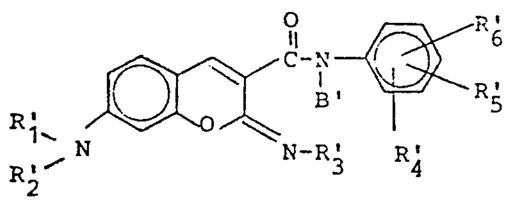

(2)

in welcher B' ein Wasserstoffatom oder eine $Alkyl_{C_1-C_4}$ gruppe, $R_1'$ und $R_2'$ unabhängig voneinander Wasserstoffatome, $Alkyl_{C_1-C_4}$ gruppen, welche durch Chlor- oder Bromatome, $Alkoxy_{C_1-C_4}$, Cyan, Hydroxyl-, Acetoxy-, Propionyloxy- oder Phenoxygruppen substituiert sein können, oder Allyl-, Cyclopentyl-, Cyclohexyl-, Phenyl-, Benzyl- oder Phenäthylgruppen bedeuten, oder zusammen mit dem Stickstoffatom einen Pyrrolidin-, Piperidin-, Pyrazolin-, Piperazin- oder Morpholinring bilden, $R_3'$ ein Wasserstoffatom oder eine Phenyl-, $—CO\text{-}Alkyl_{C_1-C_4}$, $—CO\text{-}Alkoxy_{C_1-C_4}$, —CO-Phenyl-, —CO-Cyclohexyloxy-, —CO-Phenoxy-, —CO-CH = CH-Phenyl-, $—CO\text{-}NH\text{-}Alkyl_{C_1-C_4}$, —CO-NH-Phenyl-, $—SO_2\text{-}Alkyl_{C_1-C_4}$ oder $—SO_2$-Phenyl-gruppe bedeuten, welche im Alkyl-, Alkoxy- oder Phenylrest durch Fluor-, Chlor- oder Bromatome oder Trifluormethyl-, $Alkyl_{C_1-C_4}$, $Alkoxy_{C_1-C_4}$, Cyan- oder Nitrogruppen substituiert sein können, und $R_4'$, $R_5'$ und $R_6'$ unabhängig voneinander Wasserstoff-, Fluor-, Chlor- oder Bromatome, $Alkyl_{C_1-C_4}$, $Alkoxy_{C_1-C_4}$, Trifluormethyl-, Cyano-, Nitro-, $—N(Alkyl_{C_1-C_4})_2$, $—\underset{\underset{O}{\|}}{C}\text{-}O\text{-}Alkyl_{C_1-C_4}$ oder $—SO_2\text{-}Alkyl_{C_1-C_4}$ gruppen bedeuten, und $R_4'$ und $R_5'$ zusammen, falls in o-Stellung zueinander stehend, die Gruppierung $—(CH = CH)_2—$ darstellen können.

3. Farbstoff der Formel

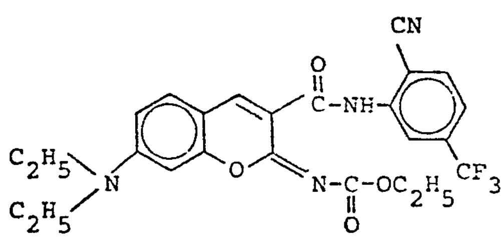

4. Farbstoff der Formel

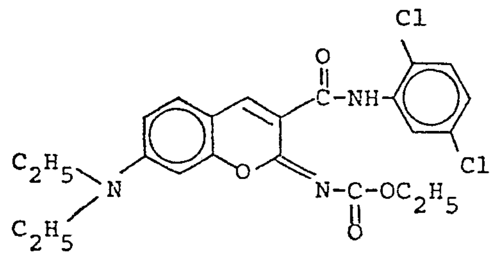

5. Farbstoff der Formel

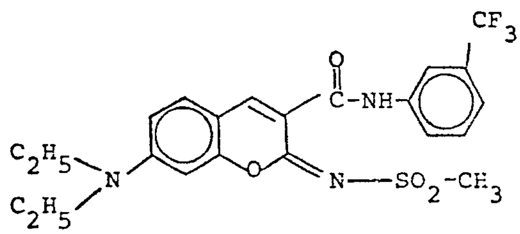

6. Farbstoff der Formel

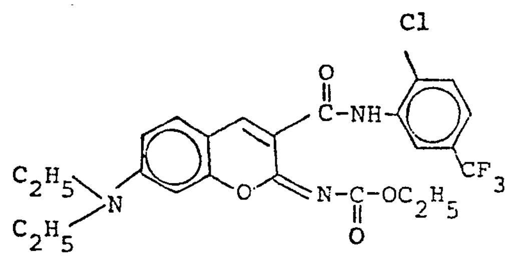

7. Farbstoff der Formel

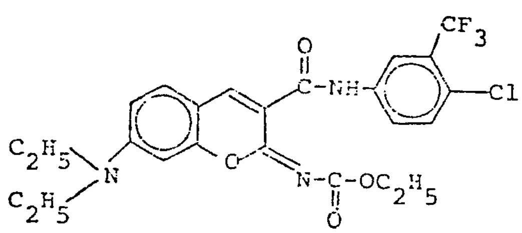

8. Verfahren zur Herstellung von wasserunlöslichen Iminocumarinfarbstoffen der allgemeinen Formel

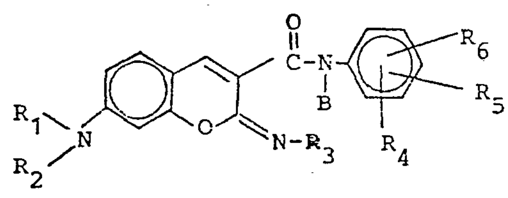

in welcher B ein Wasserstoffatom oder eine gegebenenfalls substituierte Alkylgruppe bedeutet, $R_1$ und $R_2$ unabhängig voneinander Wasserstoffatome, gegebenenfalls substituierte Alkyl- oder Arylalkylgruppen, ferner Allyl- oder Cycloalkylgruppen mit 3 bis 8 Kohlenstoffatomen, oder zusammen eine Alkylengruppe von 4 bis 5 Kohlenstoffatomen und damit zusammen mit dem Stickstoffatom einen heterocyclischen Ring, welcher weitere Heteroatome enthalten kann, bedeuten, $R_3$ ein Wasserstoffatom oder eine gegebenenfalls substituierte Aryl-, Alkylcarbonyl-, Arylcarbonyl-, Aryl-alkenylen-carbonyl-, Alkoxycarbonyl-, Aryloxycarbonyl-, Cycloalkoxycarbonyl-, Alkylcarbamoyl-, Phenylcarbamoyl-, Alkylsulfonyl- oder Arylsulfonylgruppe darstellt, $R_4$, $R_5$ und $R_6$ unabhängig voneinander Wasserstoff- oder Halogenatome, gegebenenfalls substituierte Alkyl-, Alkoxy-, Carbonsäurealkylester-, Sulfonylalkyl-, Dialkylamino-, Fluoralkylgruppen, ferner Cyan- oder Nitrogruppen bedeuten, und $R_4$ und $R_5$ zusammen, falls in o-Stellung zueinander stehend, die Gruppierung —$(CH = CH)_2$— darstellen, dadurch gekennzeichnet, daß man Cyanessigarylide der allgemeinen Formel

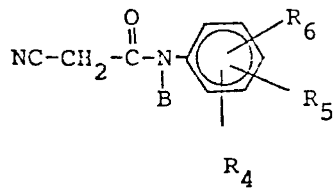

in welcher B, $R_4$, $R_5$ und $R_6$ die vorstehend genannten Bedeutungen haben, mit 4-Aminosalicylaldehyden der allgemeinen Formel

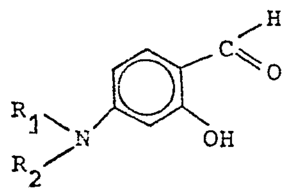

oder deren Estern mit aliphatischen Carbonsäuren mit 1-4 Kohlenstoffatomen, in welcher $R_1$ und $R_2$ die vorstehend genannten Bedeutungen haben, kondensiert und die erhaltenen Iminocumarine der allgemeinen Formel

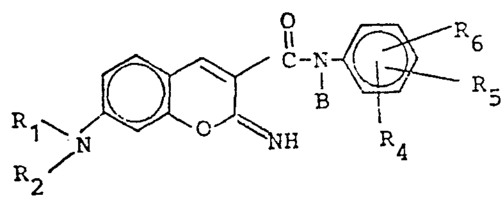

in welcher B, $R_1$, $R_2$, $R_4$, $R_5$ und $R_6$ die vorstehend genannten Bedeutungen haben, mit einem Carbonsäurehalogenid der allgemeinen Formel

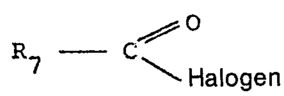

oder mit einem Carbonsäureanhydrid der allgemeinen Formel

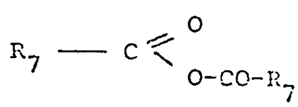

in welchen $R_7$ eine gegebenenfalls substituierte Aryl-, Alkyl-, Alkoxy- oder Aryloxygruppe bedeutet, oder mit Chlorameisensäure-alkyl$_{C_1-C_4}$, -cycloalkyl$_{C_5-C_6}$ oder -phenylester, oder mit einem Sulfonsäurehalogenid der allgemeinen Formel

$R_8$—$SO_2$—Halogen

in Gegenwart einer Base acyliert, oder mit einem Isocyanat der allgemeinen Formel

$R_8$—NCO,

worin $R_8$ eine gegebenenfalls substituierte Alkyl- oder Arylgruppe bedeutet, oder mit einem aromatischen Amin der allgemeinen Formel

$H_2N$—Aryl,

worin die Arylgruppe substituiert sein kann, umsetzt.

9. Verfahren zur Herstellung von wasserunlöslichen Iminocumarinfarbstoffen der allgemeinen Formel

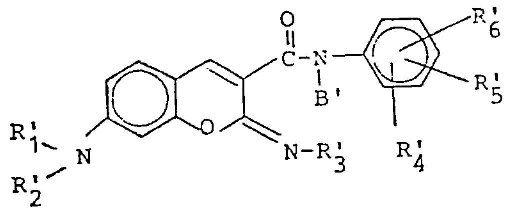

in welcher B′ ein Wasserstoffatom oder eine Alkyl$_{C_1-C_4}$ gruppe, $R_1'$ und $R_2'$ unabhängig voneinander Wasserstoffatome, Alkyl$_{C_1-C_4}$ gruppen, welche durch Chlor- oder Bromatome, Alkoxy$_{C_1-C_4}$, Cyan-, Hydroxyl-, Acetoxy-, Propionyloxy- oder Phenoxygruppen substituiert sein können, oder Allyl-, Cyclopentyl-, Cyclohexyl-, Phenyl-, Benzyl- oder Phenäthylgruppen bedeuten, oder zusammen mit dem Stickstoffatom einen Pyrrolidin-, Piperidin-, Pyrazolin-, Piperazin- oder Morpholinring bilden, $R_3'$ ein Wasserstoffatom oder eine Phenyl-, —CO-Alkyl$_{C_1-C_4}$, —CO-Alkoxy$_{C_1-C_4}$, $-\underset{\underset{O}{\|}}{C}$-Cyclohexyloxy-, —CO-Phenyl-, —CO-Phenoxy-, —CO-CH = CH-Phenyl-, —CO-NH-Alkyl$_{C_1-C_4}$, —CO-NH-Phenyl-, —$SO_2$-Alkyl$_{C_1-C_4}$ oder —$SO_2$-Phenylgruppe bedeuten, welche im Alkyl-, Alkoxy- oder Phenylrest durch Fluor-, Chlor- oder Bromatome oder Trifluormethyl-, Alkyl$_{C_1-C_4}$, Alkoxy$_{C_1-C_4}$, Cyan- oder Nitrogruppen substituiert sein können, und $R_4'$, $R_5'$ und $R_6'$ unabhängig voneinander Wasserstoff-, Fluor-, Chlor- oder Bromatome, Alkyl$_{C_1-C_4}$, Alkoxy$_{C_1-C_4}$, Trifluormethyl-, Cyano-, Nitro-, —N(Alkyl$_{C1-C4}$)$_2$, $-\underset{\underset{O}{\|}}{C}$-O-Alkyl$_{C1-C4}$ oder —$SO_2$-Alkyl$_{C_1-C_4}$ gruppen bedeuten, und $R_4'$ und $R_5'$ zusammen, falls in o-Stellung zueinander stehend, die Gruppierung —(CH = CH)$_2$— darstellen können, dadurch gekennzeichnet, daß man Cyanessigarylide der allgemeinen Formel

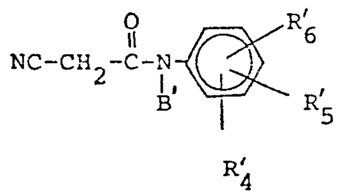

in welcher B′, $R_4'$, $R_5'$ und $R_6'$ die vorstehend genannten Bedeutungen haben, mit 4-Aminosalicylaldehyden der allgemeinen Formel

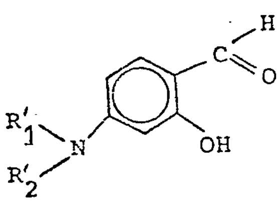

oder deren Estern mit aliphatischen Carbonsäuren mit 1-4 Kohlenstoffatomen, in welchen $R_1'$ und $R_2'$ die vorstehend genannten Bedeutungen haben, kondensiert und die erhaltenen Iminocumarine der allgemeinen Formel

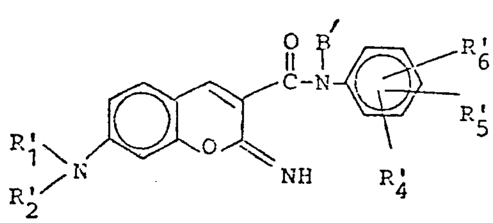

in welcher B, $R_1'$, $R_2'$, $R_4'$, $R_5'$ und $R_6'$ die vorstehend genannten Bedeutungen haben, mit einem Carbonsäurechlorid der allgemeinen Formel

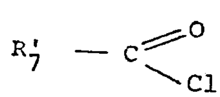

oder mit einem Carbonsäureanhydrid der allgemeinen Formel

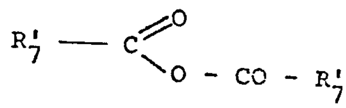

in welchen $R_7'$ eine $Alkyl_{C_1-C_4}$, $Alkoxy_{C_1-C_4}$, Phenyl- oder Phenoxygruppe bedeutet, welche im Alkyl-, Alkoxy- oder Phenylrest durch Fluor-, Chlor- oder Bromatome oder Trifluormethyl-, $Alkyl_{C_1-C_4}$, $Alkoxy_{C_1-C_4}$, Cyan- oder Nitrogruppen substituiert sein können, oder mit Chlorameisensäure-$alkyl_{C_1-C_4}$, -cyclohexyl- oder -phenylester oder mit einem Sulfonsäurechlorid der allgemeinen Formel

$$R_8'—SO_2—Cl$$

in Gegenwart einer Base acyliert, oder mit einem Isocyanat der allgemeinen Formel

$$R_8'—NCO,$$

worin $R_8'$ eine $Alkyl_{C_1-C_4}$ oder Phenylgruppe bedeutet, welche durch Fluor-, Chlor- oder Bromatome oder Trifluormethyl-, $Alkyl_{C_1-C_4}$, $Alkoxy_{C_1-C_4}$, Cyan- od. Nitrogruppen substituiert sein können, oder mit einem aromatischen Amin der allgemeinen Formel

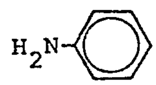

in welcher der Phenylrest durch Fluor-, Chlor- oder Bromatome, Trifluormethylgruppen, $Alkyl_{C_1-C_4}$, $Alkoxy_{C_1-C_4}$, Cyan- oder Nitrogruppen substituiert sein kann, umsetzt.

10. Fasermaterialien aus Cellulosedi-, Cellulose-2 1/2- oder Cellulosetriacetat, Polyamiden, Polyurethanen, Polycarbonaten oder Polyestern, die mit den in Ansprüchen 1 bis 7 beschriebenen Farbstoffen gefärbt oder bedruckt worden sind.

11. Polyestermaterialien, insbesondere solche aus Polyäthylenterephthalat, oder Polyamide, die mit den in Ansprüchen 1-7 beschriebenen Farbstoffen schmelzspinngefärbt wurden.

**Claims**

1. Water-insoluble iminocoumarin dyestuffs of the general formula (1)

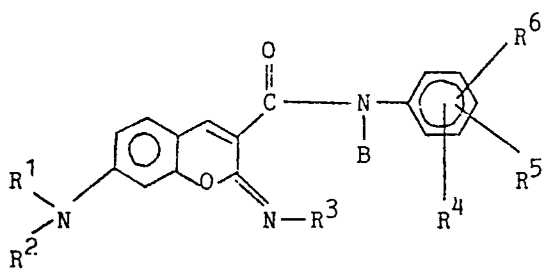

(1)

in which B represents hydrogen or optionally substituted alkyl, $R^1$ and $R^2$, independently of each other, stand for hydrogen or optionally substituted alkyl or arylalkyl, or allyl or cycloalkyl having from 3 to 8 carbon atoms, or together denote alkylene having from 4 to 5 carbon atoms and form with the nitrogen atom a heterocyclic ring possibly containing further hetero atoms, $R^3$ denotes hydrogen or optionally substituted aryl, alkylcarbonyl, arylcarbonyl, aryl-alkylene-carbonyl, alkoxycarbonyl, cycloalkoxycarbonyl, aryloxycarbonyl, alkyl-carbamoyl, phenyl-carbamoyl, alkylsulfonyl or arylsulfonyl, $R^4$, $R^5$ and $R^6$, independently of one another, represent hydrogen, halogen, optionally substituted alkyl, alkoxy, carboxylic acid alkyl ester radicals, sulfonylalkyl, dialkylamino, fluoroalkyl, cyano or nitro and $R^4$ and $R^5$ represent together the grouping $—(CH=CH)_2—$ provided that they are in ortho position with respect to each other.

2. Water-insoluble iminocoumarin dyestuffs of the general formula (2)

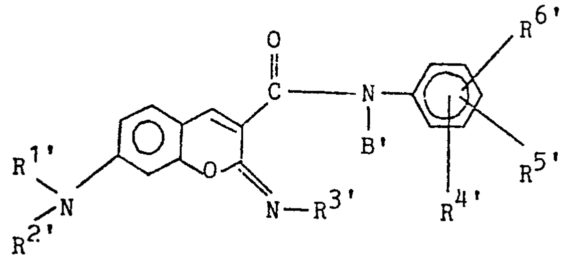

(2)

in which B' is hydrogen or $C_1$-$C_4$-alkyl, $R^{1'}$ and $R^{2'}$, independently of each other, are hydrogen, $C_1$-$C_4$

alkyl which is optionally substituted by chlorine or bromine, $C_1$-$C_4$-alkoxy, cyano, hydroxy, acetoxy, propionyloxy or by phenoxy, or are allyl, cyclopentyl, cyclohexyl, phenyl, benzyl or phenylethyl or, together with the nitrogen atom, they denote a pyrrolidine, piperidine, pyrazoline, piperazine or morpholine ring, $R^{3'}$ stands for hydrogen or phenyl, —CO-$C_1$-$C_4$-alkyl, —CO-$C_1$-$C_4$-alkoxy, —CO-cyclohexyloxy-, —CO-phenyl-, —CO-phenoxy-, —CO—CH = CH-phenyl, —CO—NH-$C_1$-$C_4$-alkyl, —CO—NH-phenyl-, —$SO_2$-$C_1$-$C_4$-alkyl- or —$SO_2$-phenyl-, in which the alkyl, alkoxy or phenyl radical may be substituted by fluorine, chlorine, bromine, trifluoromethyl, $C_1$-$C_4$-alkyl, $C_1$-$C_4$-alkoxy, cyano or nitro, $R^{4'}$, $R^{5'}$ and $R^{6'}$, independently of one another, denote hydrogen, fluorine, chlorine or bromine, $C_1$-$C_4$-alkyl, $C_1$-$C_4$-alkoxy, trifluoromethyl, cyano, nitro, —N($C_1$-$C_4$-alkyl$)_2$, $-\underset{\underset{O}{\|}}{C}-O-C_1-C_4$-alkyl, or —$SO_2$-$C_1$-$C_4$-alkyl,

and $R^{4'}$ and $R^{5'}$ together represent the grouping —$(CH = CH)_2$—provided that they are in ortho position with respect to each other.

3. Dyestuff of the formula

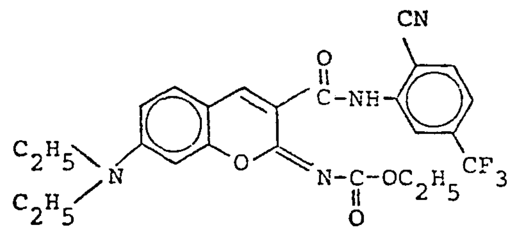

4. Dyestuff of the formula

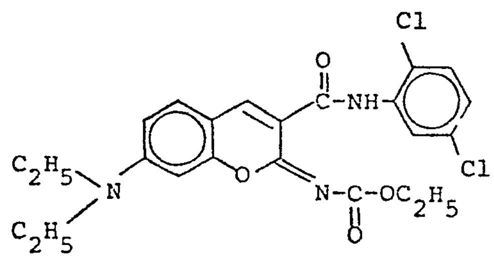

5. Dyestuff of the formula

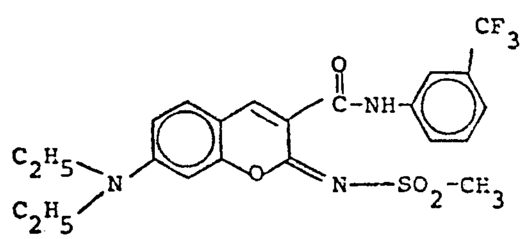

6. Dyestuff of the formula

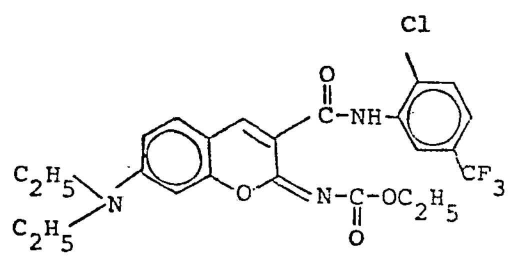

7. Dyestuff of the formula

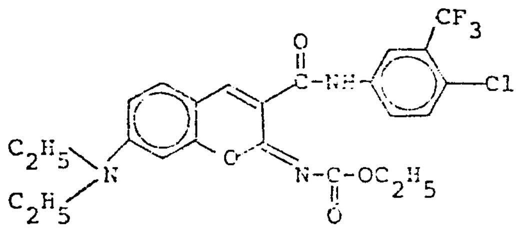

8. Process for the preparation of water-insoluble iminocoumarin dyestuffs of the general formula

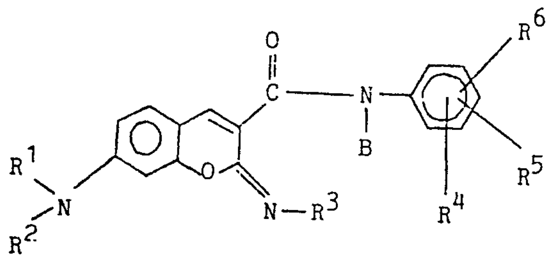

wherein B represents hydrogen or optionally substituted alkyl, $R^1$ and $R^2$, independently of each other, stand for hydrogen or optionally substituted alkyl or arylalkyl, or allyl or cycloalkyl having from 3 to 8 carbon atoms, or together denote alkylene having from 4 to 5 carbon atoms and form with the nitrogen atom a heterocyclic ring possibly containing further hetero atoms, $R^3$ denotes hydrogen or optionally substituted aryl, alkylcarbonyl, arylcarbonyl, aryl-alkylene-carbonyl, alkoxycarbonyl, cycloalkoxycarbonyl, aryloxycarbonyl, alkyl-carbamoyl, phenyl-carbamoyl, alkylsulfonyl or arylsulfonyl, $R^4$, $R^5$ and $R^6$, independently of one another, represent hydrogen, halogen, optionally substituted alkyl, alkoxy, carboxylic acid alkyl ester radicals, sulfonylalkyl, dialkylamino, fluoroalkyl, cyano or nitro and $R^4$ and $R^5$ represent together the grouping $-(CH = CH)_2-$ provided that they are in ortho position with respect to each other, which comprises condensing cyanoacetoarylides of the general formula

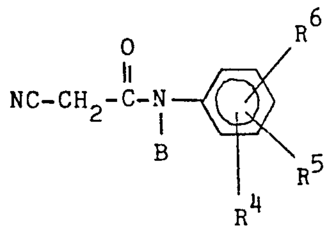

in which B, $R^4$, $R^5$ and $R^6$ are defined as above with 4-aminosalicylic aldehydes of the general formula

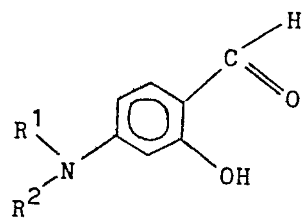

or esters thereof with aliphatic carboxylic acids having from 1 to 4 carbon atoms in which $R^1$ and $R^2$ are defined as above and acylating, in the presence of a base, the iminocoumarins obtained of the general formula

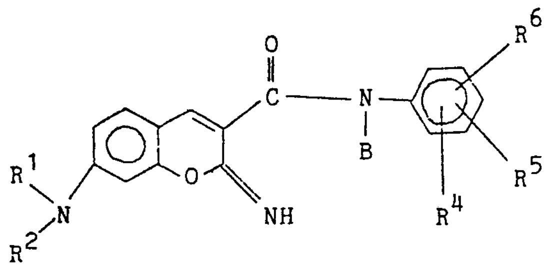

in which B, $R^1$, $R^2$, $R^4$, $R^5$ and $R^6$ are defined as above, with a carboxylic acid halide of the general formula

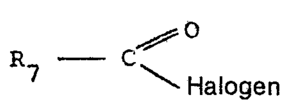

of with a carboxylic acid anhydride of the general formula or with a carboxylic acid anhydride of the formula

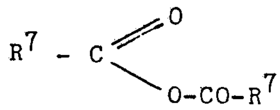

in which formulae $R^7$ represents optionally substituted aryl, alkyl, alkoxy, or aryloxy, or with a chloroformic acid $C_1$-$C_4$-alkyl ester, a chloroformic acid $C_5$-$C_6$-cycloalkyl ester or a chloroformic acid phenyl ester, or with a sulfonic acid halide of the general formula

$R^8$—$SO_2$—halogen

or reacting the iminocoumarin with an isocyanate of the general formula

$R^8$—NCO,

in which formulae $R^8$ denotes optionally substituted alkyl or aryl or with an aromatic amine of the general formula

$H_2N$—aryl,

in which the aryl radical may be substituted.

9. Process for the preparation of water-insoluble iminocoumarin dyestuffs of the general formula

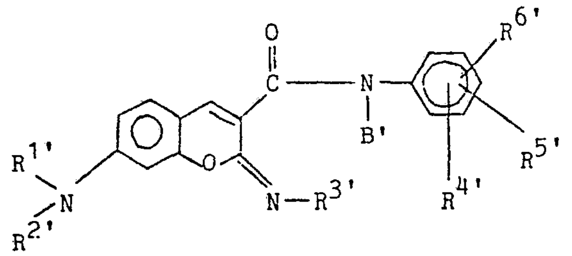

in which B′ is hydrogen or $C_1$-$C_4$-alkyl, $R^{1'}$ and $R^{2'}$, independently of each other, are hydrogen, $C_1$-$C_4$-alkyl which is optionally substituted by chlorine or bromine, $C_1$-$C_4$-alkoxy, cyano, hydroxy, acetoxy, propionyloxy or by phenoxy, or are allyl, cyclopentyl, cyclohexyl, phenyl, benzyl or phenylethyl or, together with the nitrogen atom, they denote a pyrrolidine, piperidine, pyrazoline, piperazine or morpholine ring, $R^{3'}$ stands for hydrogen or phenyl, —CO-$C_1$-$C_4$-alkyl, —CO-$C_1$-$C_4$-alkoxy, $-\underset{\mathrm{O}}{\overset{}{\mathrm{C}}}$O-cyclohexyloxy-, —CO-phenyl-, —CO-phenoxy-, —CO—CH = CH-phenyl, —CO—NH-$C_1$-$C_4$-alkyl, —CO—NH-phenyl-, —$SO_2$-$C_1$-$C_4$-alkyl- or —$SO_2$-phenyl-, in which the alkyl, alkoxy or phenyl radical may be substituted by fluorine, chlorine, bromine, trifluoromethyl, $C_1$-$C_4$-alkyl, $C_1$-$C_4$-alkoxy, cyano or nitro, $R^{4'}$, $R^{5'}$ and $R^{6'}$, independently of one another, denote hydrogen, fluorine, chlorine or bromine, $C_1$-$C_4$-alkyl, $C_1$-$C_4$-alkoxy, trifluoromethyl, cyano, nitro, —N($C_1$-$C_4$-alkyl)$_2$, $-\underset{\mathrm{O}}{\mathrm{C}}$—O-$C_1$-$C_4$-alkyl, or —$SO_2$-$C_1$-$C_4$-alkyl, and $R^{4'}$ and $R^{5'}$ together represent the grouping —(CH = CH)$_2$— provided that they are in ortho position with respect to each other, which comprises condensing cyano-acetic arylides of the general formula

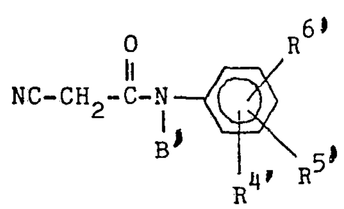

in which B′, $R^{4'}$, $R^{5'}$ and $R^{6'}$ are defined as above with 4-aminosalicylic aldehydes of the general formula

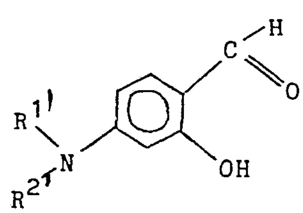

or esters thereof with aliphatic carboxylic acids having from 1 to 4 carbon atoms, in which $R^{1'}$ and $R^{2'}$

are defined as above and acylating the iminocoumarins obtained of the general formula

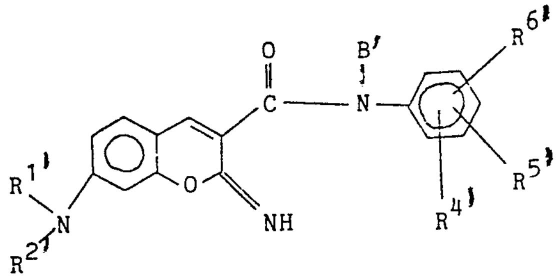

obtained in which B', $R^{1'}$, $R^{2'}$, $R^{4'}$, $R^{5'}$ and $R^{6'}$ are as defined above with a carboxylic acid chloride of the general formula

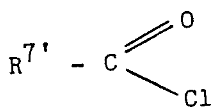

or with a carboxylic anhydride of the general formula

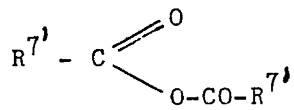

in which $R^{7'}$ denotes $C_1$-$C_4$-alkyl, $C_1$-$C_4$-alkoxy, phenyl, or phenoxy which may be substituted in the alkyl, alkoxy or phenyl radical by fluorine, chlorine, bromine, trifluoromethyl, $C_1$-$C_4$-alkyl, $C_1$-$C_4$-alkoxy, cyano or nitro, or with a chloroformic acid $C_1$-$C_4$-alkyl ester, a chloroformic acid cyclohexyl ester or a chloroformic acid phenyl ester or with a sulfonic acid chloride of the general formula

$$R^{8'}—SO_2—Cl$$

in the presence of a base, or by reacting the iminocoumarin with an isocyanate of the general formula

$$R^{8'}—NCO,$$

in which $R^{8'}$ denotes $C_1$-$C_4$-alkyl or phenyl which may be substituted by fluorine, chlorine, bromine, trifluoromethyl, $C_1$-$C_4$-alkyl, $C_1$-$C_4$-alkoxy, cyano or nitro, or with an aromatic amine of the general formula

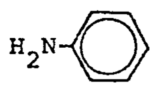

in which the phenyl radical may be substituted by fluorine, chlorine, bromine, trifluoromethyl, $C_1$-$C_4$-alkyl, $C_1$-$C_4$-alkoxy, cyano or nitro.

10. Fiber materials consisting of cellulose diacetate, cellulose 2 1/2 acetate or cellulose triacetate, of polyamides, polyurethanes, polycarbonates or polyesters which have been dyed or printed with the dyestuffs described in claims 1 to 7.

11. Polyester fiber materials, in particular those consisting of polyethylene terephthalate, or polyamides which have been dyed in a melt spinning process with the dyestuffs described in claims 1 to 7.

**Revendications**

1. Colorants imino-coumariniques insolubles dans l'eau qui répondent à la formule générale (1)

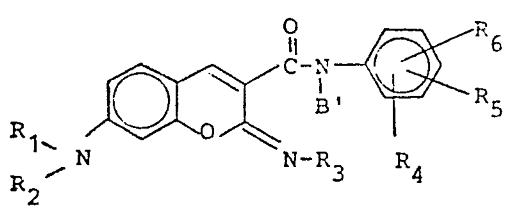

(1)

dans laquelle B représente un atome d'hydrogène ou un radical alkyle éventuellement substitué, $R_1$ et $R_2$ représentent chacun, indépendamment l'un de l'autre, un atome d'hydrogène, un radical alkyle ou arylalkyle éventuellement substitué, un radical allyle ou un radical cycloalkyle contenant de 3 à 8 atomes

de carbone, ou représentent ensemble un radical alkylène contenant 4 ou 5 atomes de carbone et forment ainsi, ensemble et avec l'atome d'azote, un noyau hétérocyclique, lequel peut contenir d'autres hétéroatomes, $R_3$ représente un atome d'hydrogène ou un radical aryle, alkylcarbonyle, arylcarbonyle, arylalcénylène-carbonyle, alcoxy-carbonyle, cycloalcoxy-carbonyle, aryloxycarbonyle, alkylcarbamoyle, phényl-carbamoyle, alkylsulfonyle ou arylsulfonyle, éventuellement substitué, $R_4$, $R_5$ et $R_6$ représentent chacun, indépendamment les uns des autres, un atome d'hydrogène ou d'halogène, un radical alkyle, alcoxy, alcoxy-carbonyle, alkyl-sulfonyle, dialkylamino ou fluoro-alkyle, éventuellement substitué, ou un groupe cyano ou nitro, et $R_4$ et $R_5$ peuvent, lorsqu'ils sont en position ortho l'un par rapport à l'autre, représenter le groupement $—(CH = CH)_2—$.

2. Colorants imino-coumariniques, insolubles dans l'eau, qui répondent à la formule générale (2)

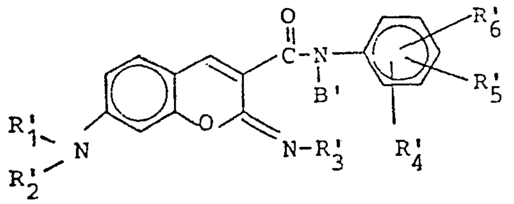

(2)

dans laquelle B' représente un atome d'hydrogène ou un alkyle en $C_1$-$C_4$, $R'_1$ et $R_2'$ représentent chacun, indépendamment l'un de l'autre, un atome d'hydrogène, un alkyle en $C_1$-$C_4$ éventuellement porteur d'un atome de chlore ou de brome ou d'un radical alcoxy en $C_1$-$C_4$, cyano, hydroxy, acétoxy, propionyloxy ou phénoxy, ou un radical allyle, cyclopentyle, cyclohexyle, phényle, benzyle ou phényl-éthyle, ou encore forment ensemble et avec l'atome d'azote un noyau de pyrrolidine, de pipéridine, de pyrazoline, de pipérazine ou de morpholine, $R_3'$ représente un atome d'hydrogène ou un phényle, un alkylcarbonyle contenant de 1 à 4 C dans sa partie alkyle, un alcoxycarbonyle à alcoxy en $C_1$-$C_4$, un phényl-carbonyle, un cyclohexyloxycarbonyle, un phénoxy-carbonyle, un styrylcarbonyle (c'est-à-dire -CO—CH = CH-phényl), un alkylamino-carbonyle à alkyle en $C_1$-$C_4$, un phénylamino-carbonyle, un alkyl-sulfonyle en $C_1$-$C_4$ ou un phényl-sulfonyle, lesquels peuvent porter, dans les radicaux alkyles, alcoxy ou phényles, un atome de fluor, de chlore ou de brome, un trifluorométhyle, un alkyle en $C_1$ à $C_4$, un alcoxy en $C_1$-$C_4$, un groupe cyano ou un groupe nitro, $R_4'$, $R_5'$ et $R_6'$ représentent chacun, indépendamment les uns des autres, un atome d'hydrogène, de fluor, de chlore ou de brome, un alkyle en $C_1$-$C_4$, un alcoxy en $C_1$-$C_4$, un trifluorométhyle, un cyano, un nitro, un radical $-N(alkyl_{C1-C4})_2$ un radical alcoxycarbonyle à alkyle en $C_1$-$C_4$ ou un alkylsulfonyle en $C_1$-$C_4$, et $R_4'$ et $R_5'$ peuvent former ensemble, lorsqu'ils sont en position ortho l'un par rapport à l'autre, le groupement $—(CH = CH)_2—$.

3. Colorant de formule

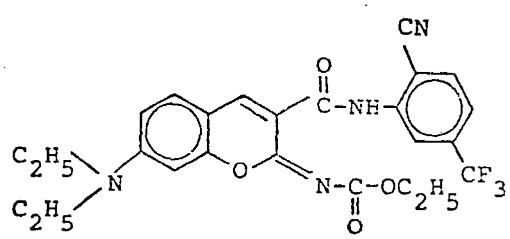

4. Colorant de formule

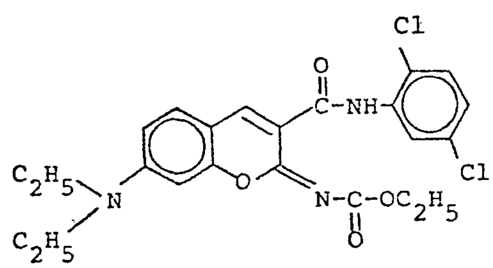

5. Colorant de formule

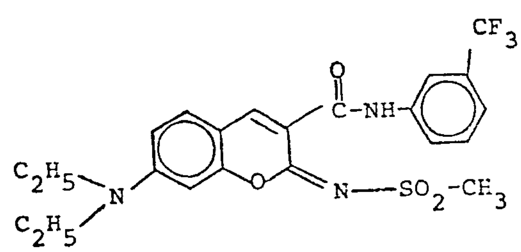

6. Colorant de formule

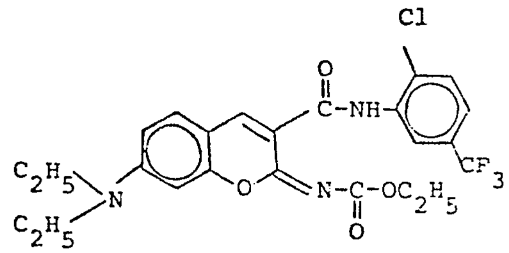

7. Colorant de formule

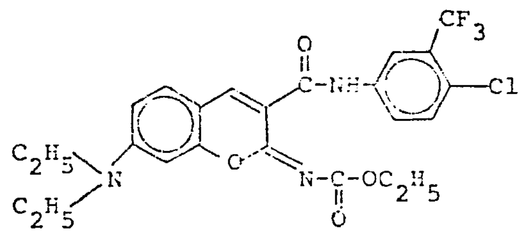

8. Procédé pour préparer des colorants imino-coumariniques insolubles dans l'eau qui répondent à la formule générale

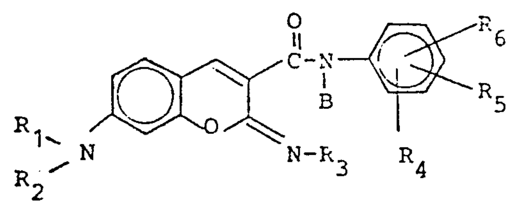

dans laquelle B représente un atome d'hydrogène ou un radical alkyle éventuellement substitué, $R_1$ et $R_2$ représentent chacun, indépendamment l'un de l'autre, un atome d'hydrogène, un radical alkyle ou arylalkyle éventuellement substitué, un radical allyle ou un radical cycloalkyle contenant de 3 à 8 atomes de carbone, ou représentent ensemble un radical alkylène contenant 4 ou 5 atomes de carbone et forment ainsi ensemble et avec l'atome d'azote, un noyau hétérocyclique, lequel peut contenir d'autres hétéroatomes, $R_3$ représente un atome d'hydrogène ou un radical aryle, alkylcarbonyle, arylcarbonyle, arylalcénylène-carbonyle, alcoxy-carbonyle, cycloalcoxy-carbonyle, aryloxycarbonyle, alkylcarbamoyle, phényl-carbamoyle, alkyl-sulfonyle ou arylsulfonyle, éventuellement substitué, $R_4$, $R_5$ et $R_6$ représentent chacun, indépendamment les uns des autres, un atome d'hydrogène ou d'halogène, un radical alkyle, alcoxy, alcoxy-carbonyle alkyl-sulfonyle, dialkylamino ou fluoro-alkyle, éventuellement substitué, ou un groupe cyano ou nitro, et $R_4$ et $R_5$ peuvent, lorsqu'ils sont en position l'un par rapport à l'autre, représenter le groupement $-(CH = CH)_2-$, procédé caractérisé en ce qu'on condense un cyanacétarylide répondant à la formule générale

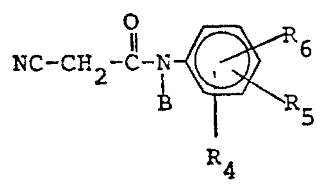

dans laquelle B, $R_4$, $R_5$ et $R_6$ ont les significations précédemment données, avec un amino-4 salicylaldéhyde répondant à la formule générale

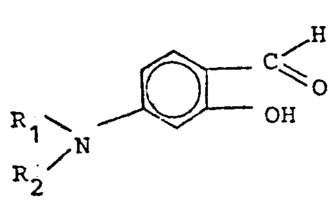

(ou l'un de ses esters d'acides carboxyliques aliphatiques en $C_1$-$C_4$) dans laquelle $R_1$ et $R_2$ ont les significations précédemment données, et on acyle l'imino-coumarine obtenue, qui répond à la formule générale

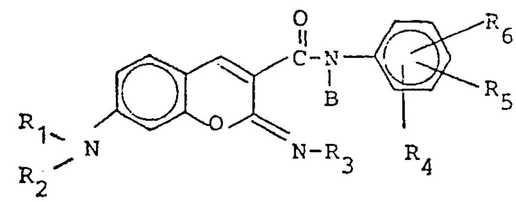

dans laquelle B, $R_1$, $R_2$, $R_4$, $R_5$ et $R_6$ ont les significations précédemment données, avec un halogénure d'acide carboxylique de formule générale

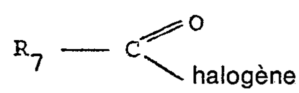

ou avec un anhydride d'acide carboxylique de formule 15 générale

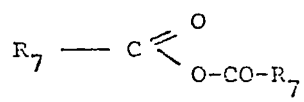

formules dans lesquelles $R_7$ représente un radical aryle, alkyle, alcoxy ou aryloxy, éventuellement substitué, ou avec un chloroformiate d'alkyle en $C_1$-$C_4$, de cycloalkyle en $C_5$ ou $C_6$ ou de phényle, ou avec un halogénure d'acide sulfonique de formule générale

$$R_8—SO_2—\text{halogène}$$

en présence d'une base, ou on fait réagir ladite imino-coumarine avec un isocyanate répondant à la formule générale

$$R_8—NCO$$

dans laquelle $R_8$ représente un radical alkyle ou aryle éventuellement substitué, ou avec une amine aromatique répondant à la formule générale

$$H_2N—\text{aryl}$$

dans laquelle le radical aryle peut être substitué.

9. Procédé de préparation de colorants imino-coumariniques insolubles dans l'eau qui répondent à la formule générale

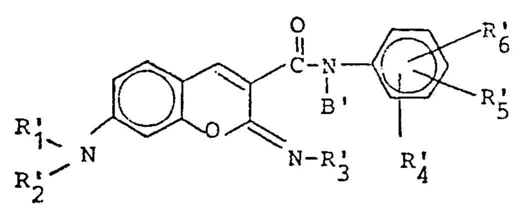

dans laquelle B' représente un atome d'hydrogène ou un alkyle en $C_1$-$C_4$, $R_1'$ et $R_2'$ représentent chacun, indépendamment l'un de l'autre, un atome d'hydrogène, un alkyle en $C_1$-$C_4$ éventuellement porteur d'un atome de chlore ou de brome ou d'un radical alcoxy en $C_1$-$C_4$, cyano, hydroxy, acétoxy, propionyloxy ou phénoxy, ou un radical allyle, cyclopentyle, cyclohexyle, phényle, benzyle ou phényléthyle, ou encore forment ensemble et avec l'atome d'azote un noyau de pyrrolidine, de pipéridine, de pyrazoline, de pipérazine ou de morpholine, $R_3'$ représente un atome d'hydrogène ou un phényle, un alkylcarbonyle contenant de 1 à 4 C dans sa partie alkyle, un alcoxycarbonyle à alcoxy en $C_1$-$C_4$, un phényl-carbonyle, un cyclohexyloxycarbonyle, un phénoxy-carbonyle, un styrylcarbonyle (c'est-à-dire -CO-CH = CH-phényl), un alkylamino-carbonyle à alkyle en $C_1$-$C_4$, un phénylamino-carbonyle, un alkylsulfonyle en $C_1$-$C_4$ ou un phényl-sulfonyle, lesquels peuvent porter, dans les radicaux alkyles, alcoxy ou phényles, un atome de fluor, de chlore ou de brome, un trifluorométhyle, un alkyle en $C_1$ à $C_4$, un alcoxy en $C_1$-$C_4$, un groupe cyano ou un groupe nitro, $R_4'$, $R_5'$ et $R_6'$ représentent chacun, indépendamment les uns des autres, un atome d'hydrogène, de fluor, de chlore ou de brome, un alkyle en $C_1$-$C_4$, un alcoxy en $C_1$-$C_4$, un trifluorométhyle, un cyano, un nitro, un radical $-N(alkyl_{C_1-C_4})_2$, un radical alcoxycarbonyle à alkyle en $C_1$-$C_4$ ou un alkylsulfonyle en $C_1$-$C_4$, et $R_4'$ et $R_5'$ peuvent former ensemble, lorsqu'ils sont en position ortho l'un par rapport à l'autre, le groupement $—(CH = CH)_2—$, procédé caractérisé en ce

qu'on condense un cyanacétarylide répondant à la formule générale

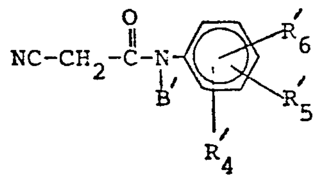

dans laquelle B', $R_4'$, $R_5'$ et $R_6'$ ont les significations précédemment données, avec un amino-4 salicylaldéhyde répondant à la formule générale

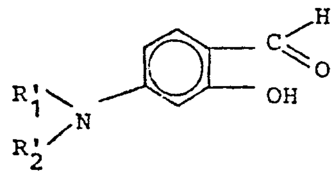

(ou l'un de ses esters d'acides carboxyliques aliphatiques en $C_1$-$C_4$) dans laquelle $R_1'$ et $R_2'$ ont les significations précédemment données, et on acyle l'imino-coumarine obtenue, qui répond à la formule générale

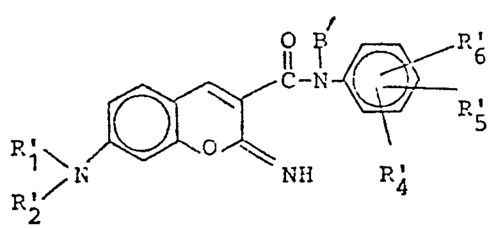

dans laquelle B, $R_1'$, $R_2'$, $R_4'$, $R_5'$ et $R_6'$ ont les significations précédemment données, avec un chlorure d'acide carboxylique de formule générale

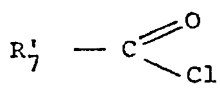

ou avec un anhydride d'acide carboxylique de formule générale

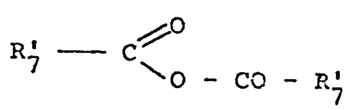

formules dans lesquelles $R_7'$ représente un radical alkyle en $C_1$-$C_4$, un radical alcoxy en $C_1$-$C_4$, un radical phényle ou un radical phénoxy, radicaux dans lesquels la partie alkyle, alcoxy ou phényle peut porter un atome de fluor, de chlore ou de brome ou un radical trifluorométhyle, alkyle en $C_1$-$C_4$, alcoxy en $C_1$-$C_4$, cyano ou nitro, ou avec un chloroformiate d'alkyle en $C_1$-$C_4$, de cyclohexyle ou de phényle, ou avec un chlorure d'acide sulfonique de formule générale

$$R_8'—SO_2—Cl$$

en présence d'une base, ou on fait réagir ladite imino-coumarine avec un isocyanate répondant à la formule générale

$$R_8'—NCO,$$

formules dans lesquelles $R_6'$ représente un radical alkyle en $C_1$-$C_4$ ou un radical phényle, éventuellement porteur d'un atome de fluor, de chlore ou de brome ou d'un radical trifluorométhyle, alkyle en $C_1$-$C_4$, alcoxy en $C_1$-$C_4$, cyano ou nitro, avec une amine aromatique répondant à la formule générale

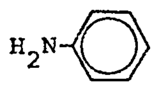 ,

dans laquelle le radical phényle peut porter un atome de fluor, de chlore ou de brome, ou un radical trifluorométhyle, alkyle en $C_1$-$C_4$, alcoxy en $C_1$-$C_4$, cyano ou nitro.

10. Matières fibreuses en diacétate de cellulose, en hémipenta-acétate de cellulose ou en tri-acétate de cellulose, en polyamides, en polyuréthannes, en polycarbonates ou en polyesters qui ont été teintes ou imprimées avec les colorants décrits dans les revendications 1 à 7.

11. Matières en polyesters, plus particulièrement en polytéréphtalate d'éthylène-glycol, ou en polyamides, qui ont été teintes lors du filage à l'état fondu avec les colorants décrits dans les revendications 1 à 7.